# EUROPÄISCHE PATENTSCHRIFT

(11) **EP 2 917 285 B1**
(45) Veröffentlichungstag und Bekanntmachung des Hinweises auf die Patenterteilung: **12.12.2018**
(21) Anmeldenummer: 13776726.5
(22) Anmeldetag: 11.10.2013
(51) Int. Cl.: C09C 1/00, A61Q 1/02, A61Q 5/02, A61Q 19/10, A61K 8/02, C09D 17/00

(54) **PIGMENTE**
PIGMENTS
PIGMENTS

(30) Priorität: 18.10.2012 DE 102012020392
(43) Veröffentlichungstag der Anmeldung: 16.09.2015
(73) Patentinhaber: Merck Patent GmbH, 64293 Darmstadt (DE)
(72) Erfinder: MELSON, Sabine, 64367 Muehltal (DE); SCHOEN, Sabine, 45701 Herten (DE)
(86) Internationale Anmeldenummer: PCT/EP2013/003063
(87) Internationale Veröffentlichungsnummer: WO 2014/060083

(56) Entgegenhaltungen:
- EP-A1- 2 607 432
- US-A1- 2010 175 587
- "Sample dispersion & refractive index guide", INTERNET CITATION, 1. April 1997 (1997-04-01), Seite Complete, XP007910412, Gefunden im Internet: URL:http://spamsnap80.engr.uc.edu/PublicSh are/MastersizerManuals/Refractive%20Index% 20Guide.pdf [gefunden am 2009-11-03]

## Beschreibung

Die vorliegende Erfindung betrifft Pigmente auf der Basis von mehrfach beschichteten plättchenförmigen Substraten, die sich dadurch auszeichnen, dass sie auf der Oberfläche mindestens 4 Schichten aufweisen, sowie deren Verwendung, u.a. in Farben, Lacken, Druckfarben, Kunststoffen und in kosmetischen Formulierungen.

Mehrschichtpigmente werden als Glanz- oder Effektpigmente in vielen Bereichen der Technik eingesetzt, insbesondere in der dekorativen Beschichtung, im Kunststoff, in Farben, Lacken, Druckfarben sowie in kosmetischen Formulierungen. Interferenzpigmente bestehen in der Regel aus plättchenförmigen Trägern, die mit dünnen Metalloxidschichten belegt sind. Die optische Wirkung dieser Pigmente beruht auf der gerichteten Reflexion von Licht an den vorwiegend parallel ausgerichteten Plättchen. Dabei entstehen durch Reflexion des Lichtes an den Grenzflächen von Schichten mit unterschiedlichem Brechungsindex Interferenzfarben (G. Pfaff in High Performance Pigments, Wiley-VCH Verlag, Weinheim, 2002, Kap. 7, Special Effect Pigments).

Aus dem Stand der Technik sind Verfahren zur Herstellung von Mehrschichtpigmenten bekannt, mit deren Hilfe alternierende Schichten mit hoher und niedriger Brechzahl auf feinteilige Substrate aufgebracht werden können. Derartige Pigmente auf Basis von mehrfach beschichteten plättchenförmigen Substraten sind z. B. aus der U.S. 4,434,010, JP H7-759, U.S. 3,438,796, U.S. 5,135,812, DE 44 05 494, DE 44 37 753, DE 195 16 181 und DE 195 15 988, DE 196 18 565, DE 197 46 067, US 2010/175587 A1 und EP 2 607 432 A1 sowie aus der Literatur z.B. aus EURO COSMETICS, 1999, Nr. 8, S. 284 bekannt.

Die aus dem Stand der Technik bekannten Mehrschichtpigmente zeigen aber den Nachteil, dass sie in der Regel zu wenig deckend sind. Insbesondere ist das Deckvermögen der Pigmente abhängig von der Partikelgröße und Partikeldicke. Fraktionen mit kleineren Partikeln, z.B. D₉₀ ≤ 40 µm bei einer Dicke von ≅ 300 nm, weisen ein höheres Deckvermögen auf als Partikel mit einer Dicke von > 300 nm und einer D₉₀ > 40 µm. Allerdings zeigen Fraktionen mit D₉₀ > 40 µm in der Regel zwar einen höheren Sparkle, aber dafür ein geringes Deckvermögen.

Aufgabe der vorliegenden Erfindung ist es daher Mehrschichtpigmente bereitzustellen, die die oben angegebenen Nachteile nicht aufweisen, und insbesondere sich durch einen hohen Sparkle-Effekt verbunden mit einem relativ hohen Deckvermögen auszeichnen.

Überraschenderweise wurden nun Mehrschichtpigmente auf der Basis plättchenförmiger Substrate gefunden, die bezüglich ihrer koloristischen aber auch ihrer anwendungstechnischen Eigenschaften deutlich verbesserte Eigenschaften, insbesondere ein relativ hohes Deckvermögen verbunden mit hoher Reflektivität und Sparkle zeigen, verglichen mit den Mehrschichtpigmenten aus dem Stand der Technik. Dies konnte durch eine innere Strukturierung von 3 hochbrechenden Schichten auf dem Substrat realisiert werden.

Gegenstand der Erfindung sind daher Mehrschichtpigmente auf der Basis von mehrfach beschichteten plättchenförmigen Substraten, die auf dem Substrat mindestens 4 Schichten [Schichten (A) - (D)] aufweisen, wobei
(A0) optional eine Schicht aus SiO₂,
(A) eine farblose Beschichtung mit einem Brechungsindex n < 1,8, wobei die Schicht (A) chemisch nicht identisch ist mit der Schicht (A0),
(B) eine farblose Beschichtung mit einem Brechungsindex n ≥ 1,8, wobei die Schicht B Dicken von 1-15 nm aufweist,
(C) eine farblose Beschichtung mit einem Brechungsindex n ≥ 1,8, wobei die Schicht (C) chemisch nicht identisch ist mit der Schicht (B),
(D) eine farbige Beschichtung mit einem Brechungsindex n ≥ 1,8 und optional
(E) eine äußere Schutzschicht,
ist.

Die erfindungsgemäßen Mehrschichtpigmente zeichnen sich gegenüber den Mehrschichtpigmenten aus dem Stand der Technik durch
- eine deutlich erhöhte Helligkeit und damit eine hohe Reflektivität,
- einen stärkeren Glanz,
- ein sehr gutes Haftvermögen auf der Haut,
- ein höheres Deckvermögen,
- eine hohe mechanische Stabilität,
- eine hohe Photostabilität, und
- eine hohe Farbstärke
aus.

Die erfindungsgemäßen Pigmente sind den Mehrschichtpigmenten aus dem Stand der Technik nicht nur hinsichtlich ihrer optischen Eigenschaften, wie insbesondere Glanz, Sparkle und Farbstärke, sondern auch in ihren anwendungstechnischen Eigenschaften, wie z. B. der mechanischen Stabilität und der Photostabilität, deutlich überlegen.

Gegenstand der Erfindung ist weiterhin die Verwendung der erfindungsgemäßen Pigmente in Farben, Lacken, insbesondere Automobillacken, Pulverlacken, Druckfarben, Sicherheitsdruckfarben, Kunststoffen, keramischen Materialien, Gläsern, Papier, in Tonern für elektrophotographische Druckverfahren, im Saatgut, in Gewächshausfolien und Zeltplanen, als Absorber bei der Lasermarkierung von Papier und Kunststoffen, in kosmetischen Formulierungen. Weiterhin sind die erfindungsgemäßen Pigmente auch zur Herstellung von Pigmentanteigungen mit Wasser, organischen und/oder wässrigen Lösemitteln, Pigmentpräparationen sowie zur Herstellung von Trockenpräparaten, wie z. B. Granulaten, Chips, Pellets, Briketts, etc., geeignet. Die Trockenpräparate sind insbesondere für Druckfarben und in der Kosmetik geeignet.

Geeignete Basissubstrate für die erfindungsgemäßen Pigmente sind farblose oder selektiv oder nicht selektiv absorbierende plättchenförmige Substrate. Geeignete Substrate sind insbesondere Schichtsilikate wie natürlicher und/oder synthetischer Glimmer, Talkum, Kaolin, plättchenförmige Eisen- oder Aluminiumoxide, Glas-, SiO₂-, TiO₂-, Graphitplättchen, synthetische trägerfreie Plättchen, Titannitrid, Titansilicid, Liquid crystal polymers (LCPs), holographische Pigmente, BiOCl und plättchenförmige Mischoxide oder deren Gemische. Besonders bevorzugte Substrate sind Glasplättchen, natürliche oder synthetische Glimmerplättchen und Al₂O₃-Plättchen.

Insbesondere bevorzugt sind Glasplättchen aufgrund ihrer besonders glatten Oberfläche und ihres sehr hohen Reflexionsvermögens, ferner synthetische Glimmerplättchen und Al₂O₃-Plättchen.

Geeignete Gläser sind alle dem Fachmann bekannten Gläser, beispielsweise Silikatgläser, wie Kalknatronglas, Borosilikatglas, Aluminosilikatglas, Bleikristallglas, E-, A-, C-, ECR-Glas, Duranglas, Fensterglas, Laborglas, etc. Derartige Gläser werden aus Sand, Kalk, Tonerde, Borverbindungen, Pottasche, Soda, usw. erschmolzen und in einem geformten Zustand erstarren gelassen. Geeignete Glasplättchen bestehen vorzugsweise aus C-, E-, ECR- oder Borosilikatglas. Es können selbstverständlich auch Gemische von verschiedenen Glasplättchen eingesetzt werden, die sich nur in der Glaszusammensetzung unterscheiden. Insbesondere bevorzugt sind Substratplättchen aus Calcium-Aluminiumborosilikat oder ECR-Glas, ferner E-, A- und C-Glas. Durch den Zusatz von anorganischen Farbmitteln können die Glasplättchen bei der Herstellung gezielt eingefärbt werden. Geeignete Farbmittel sind solche, die bei der Schmelztemperatur des Glases sich nicht zersetzen. Das Farbmittel wird in der Regel in Mengen von 0,1 - 50 Gew. %, insbesondere von 0,2 - 25 Gew.% und ganz besonders bevorzugt von 0,5 - 10 Gew. %, der Glasschmelze zugesetzt.

Geeignete Färbemittel sind insbesondere die Kationen oder komplexe Anionen der Elemente Cu, Cr, Mn, Fe und Co und/oder deren Kombinationen. Durch den Zusatz der Ionen können intensive Blau-, Grün-, Gelb-, Orange- oder Rotfärbungen erhalten werden. Geeignete Färbemittel sind weiterhin TiO₂ oder elementare Edelmetalle.

Der Brechungsindex geeigneter Glasplättchen liegt vorzugsweise bei 1,45-1,80, insbesondere bei 1,50-1,70.

In einer bevorzugten Ausführungsform wird das Substrat vor der Belegung mit den Schichten (A)-(D) mit einer Schutzschicht (Schicht (A0)) versehen. Diese Vorbehandlung empfiehlt sich insbesondere bei der Verwendung von Glasplättchen als Substrat. Diese Schutzschicht ist vorzugsweise eine SiO₂-Schicht. Die chemische Zusammensetzung der Glasplättchen ist bei einer Belegung mit einer SiO₂-Schicht (Schicht (A0)) allerdings von untergeordneter Bedeutung für die weiteren Beschichtungen und die resultierenden anwendungstechnischen Eigenschaften der Pigmente. Durch die SiO₂-Belegung wird die Glasoberfläche vor chemischer Veränderung wie Quellung, Auslaugen von Glasbestandteilen oder Auflösung in den aggressiven sauren Belegungslösungen geschützt.

Die Größe der Basissubstrate ist an sich nicht kritisch und kann auf den jeweiligen Anwendungszweck abgestimmt werden. In der Regel haben die plättchenförmigen Substrate eine Dicke zwischen 0,005 und 10 µm, insbesondere zwischen 0,1 und 2 µm. Die Ausdehnung in den beiden anderen Bereichen beträgt üblicherweise 1-500 µm, vorzugsweise 2-300 µm und insbesondere 20-200 µm. Bevorzugte kleinere Partikelgrößen sind weiterhin solche im Bereich von 1-100 µm, insbesondere 5-60 µm und 1-15 µm.

Besonders bevorzugt sind Glasplättchen mit einer durchschnittlichen Dicke von < 2 µm. Dickere Plättchen können in den gängigen Druckverfahren und bei anspruchsvollen Lackierungen in der Regel nicht eingesetzt werden. Vorzugsweise besitzen die Glasplättchen mittlere Dicken von ≤ 1 µm, insbesondere von < 0,9 µm, ganz besonders bevorzugt von < 0,7 µm. Insbesondere bevorzugt sind Glasplättchen mit Dicken von 200 - 1000 nm. Der Durchmesser der Glasplättchen liegt vorzugsweise bei 5 - 300 µm, insbesondere bevorzugt bei 10 - 300 µm. Glasplättchen mit diesen Dimensionen sind kommerziell im Handel erhältlich.

Mehrschichtpigmente basierend auf synthetischen Glimmerplättchen weisen vorzugsweise folgende Partikelgrößenverteilungen auf:
D₁₀ = 1 - 40 µm, vorzugsweise 4 - 25 µm
D₅₀ = 5 - 80 µm, vorzugsweise 8 - 55 µm
D₉₀ = 10 - 150 µm, vorzugsweise 15 - 100 µm.

Mehrschichtpigmente basierend auf natürlichen Glimmerplättchen weisen vorzugsweise folgende Partikelgrößenverteilungen auf:
D₁₀ = 1 - 15 µm, vorzugsweise 1 - 11 µm
D₅₀ = 3 - 30 µm, vorzugsweise 6 - 23 µm
D₉₀ = 5 - 80 µm, vorzugsweise 11 - 47 µm.

Mehrschichtpigmente basierend auf Glasplättchen weisen vorzugsweise folgende Partikelgrößenverteilungen auf:
D₁₀ = 10 - 50 µm, vorzugsweise 14 - 33 µm
D₅₀ = 20 - 100 µm, vorzugsweise 33 - 81 µm
D₉₀ = 50 - 200 µm, vorzugsweise 79 - 175 µm.

Die Charakterisierung der Teilchengrößenverteilung findet mittels Laserbeugung statt. In der vorliegenden Anmeldung wird die Teilchengrößenverteilung mit dem Gerät Malvern Mastersizer 2000 bestimmt.

Die erfindungsgemäßen Mehrschichtpigmente weisen folgende Schichtenfolgen auf dem Substrat auf:
(A) eine farblose Beschichtung mit einem Brechungsindex n < 1,8,
(B) eine farblose Beschichtung mit einem Brechungsindex n ≥ 1,8,
(C) eine farblose Beschichtung mit einem Brechungsindex n ≥ 1,8, wobei die Schicht (C) chemisch nicht identisch ist mit der Schicht (B),
(D) eine farbige Beschichtung mit einem Brechungsindex n ≥ 1,8 und optional
(E) eine äußere Schutzschicht.
oder
(A0) eine Schicht aus SiO₂,
(A) eine farblose Beschichtung mit einem Brechungsindex n < 1,8, wobei die Schicht (A) chemisch nicht identisch ist mit der Schicht (A0),
(B) eine farblose Beschichtung mit einem Brechungsindex n ≥ 1,8,
(C) eine farblose Beschichtung mit einem Brechungsindex n ≥ 1,8, wobei die Schicht (C) chemisch nicht identisch ist mit der Schicht (B),
(D) eine farbige Beschichtung mit einem Brechungsindex n ≥ 1,8 und optional
(E) eine äußere Schutzschicht.

Die Schicht (A) bzw. (A0) wird direkt auf die Oberfläche des Substrats appliziert.

Die Dicke der Schicht (A0), sofern auf dem Substrat vorhanden, kann in Abhängigkeit vom gewünschten Effekt in weiten Bereichen variiert werden. Die Schicht (A0) weist vorzugsweise Dicken von 3 - 150 nm, insbesondere von 5 - 100 nm, und ganz besonders bevorzugt von 10 - 50 nm, auf.

Die SiO₂-Schicht kann auch mit Rußpartikeln, anorganischen Farbpigmenten, und/oder Metallpartikeln dotiert sein, sofern diese Dotierung an Luft oder unter Inertgas bei Temperaturen > 700 °C stabil ist. Der Anteil an Dotiermittel in der SiO₂-Matrix beträgt dann 1-30 Gew.%, vorzugsweise 2-20 Gew.%, insbesondere 5-20 Gew.%.

Die niedrigbrechende Beschichtung (A), sofern vorhanden, besteht vorzugsweise aus niedrigbrechende Materialien, wie z.B. Al₂O₃, AIO(OH), B₂O₃, MgF₂, MgSiO₃ oder ein Gemisch der Verbindungen. Insbesondere bevorzugt besteht die Schicht (A) aus Al₂O₃.

Die Schicht (A) weist vorzugsweise Dicken von 1 - 50 nm, insbesondere von 1 - 30 nm, und ganz besonders bevorzugt von 1 - 15 nm, auf.

Vorzugsweise bestehen die hochbrechenden Schichten (B) und (C) aus farblosen Metalloxiden, wie z. B. TiO₂, ZrO₂, SnO₂, ZnO.

Das Titandioxid kann in der hochbrechenden Beschichtung in der Rutil- oder in der Anatasmodifikation vorliegen, vorzugsweise liegt es als Rutil vor.

Die Dicke der hochbrechenden Schichten richtet sich nach der gewünschten Interferenzfarbe.

Die Schicht (B) weist Dicken von 1 - 15 nm auf.

Die Schicht (C) weist vorzugsweise Dicken von 5 - 300 nm, insbesondere von 10 - 200 nm, und ganz besonders bevorzugt von 20 - 120 nm, auf.

Vorzugsweise handelt es sich bei der Schicht (D) um eine farbige Metalloxidschicht, die insbesondere aus Fe₂O₃, Fe₃O₄, Cr₂O₃, Fe₂TiO₅, ein Gemisch aus TiO₂ und Fe₂O₃, Ce₂O₃, CoO, Co₃O₄, VO₂, V₂O₃, NiO, ferner aus Titansuboxiden (TiO₂ teilweise reduziert mit Oxidationszahlen von < 4 bis 2 wie die niederen Oxide Ti₃O₅, Ti₂O₃ bis zu TiO), Titanoxynitriden, FeO(OH) oder Fe₂SiO₄ besteht. Besonders bevorzugt handelt es sich bei der Schicht (D) um eine Schicht aus Eisenoxid, insbesondere aus Fe₂O₃.

Vorzugsweise besitzt die Schicht (D) Dicken von 1 - 300 nm, insbesondere von 50 - 250 nm, und ganz besonders bevorzugt von 50 - 150 nm.

In Abhängigkeit von der Glühtemperatur, vorzugsweise bei Temperaturen < 750 °C, und/oder dem Substrat kann zwischen der Schicht (C) und (D) eine weitere Zwischenschicht (C0) vorhanden sein, die sich aus den Komponenten der Schichten (C) und (D) zusammensetzt. Es handelt sich um eine Mischschicht aus den Schichten (C) und (D). Die Schicht (C0), sofern vorhanden, besitzt vorzugsweise Schichtdicken von < 50 nm.

Die Dicke der einzelnen Schichten (A) - (D) mit hohem bzw. niedrigem Brechungsindex ist wichtig für die optischen Eigenschaften des Pigments. Vorzugsweise beträgt die Schichtdicke aller Schichten, d.h. Schichten (A0)-(D), auf dem Substrat 20 - 600 nm, insbesondere 100 - 350 nm.

Die Schichten (A)-(D) bestehen vorzugsweise aus Oxiden, ausgewählt aus der Gruppe Al₂O₃, TiO₂, ZrO₂, SnO₂, ZnO, Ce₂O₃, Fe₂O₃, Fe₃O₄, FeOOH, Cr₂O₃, CoO, Co₃O₄, SiO₂, VO₂, V₂O₃, NiO, Titansuboxiden oder deren Gemische.

In einer besonders bevorzugten Ausführungsform enthält die hochbrechende Beschichtung, d. h. Schichten (B), (C) und/oder (D) und optional die Schicht (C0), vorzugsweise Schicht (C) und/oder (D), als weiteren Bestandteil eine oder mehrere Erdalkalimetallverbindungen. Vorzugsweise wird eine hochbrechende Beschichtung aus TiO₂ oder Fe₂O₃ mit einem Erdalkalititanat, Erdalkalistannat und/oder Erdalkalioxid dotiert. Alternativ kann auch Zinkoxid, Al₂O₃ und/oder Al₂TiO₅ in Kombination mit oder anstelle der genannten Erdalkaliverbindungen eingesetzt werden.

In einer besonders bevorzugten Ausführungsform besteht das Erdalkalititanat in der hochbrechenden Beschichtung aus Magnesium-, Calcium-, Strontium- oder Bariumtitanat, das Erdalkalioxid aus Magnesium-, Calcium-, Strontium- oder Bariumoxid. Alternativ kann auch Aluminiumstannat und/oder Al₂O₃ eingesetzt werden. Weiterhin ist auch ein Gemisch der genannten Erdalkaliverbindungen möglich.

Ganz besonders bevorzugt sind Pigmente, deren Schicht (D) eine Fe₂O₃-Schicht ist, die mindestens mit einem Erdalkalioxid ausgewählt aus der Gruppe Magnesium, Calcium-, Strontium- oder Bariumoxid dotiert ist. Alternativ kann auch Aluminiumoxid verwendet werden. Besonders bevorzugt ist die Schicht (D) mit Calciumoxid und/oder Aluminiumoxid dotiert.

Die Dotierung beträgt in der jeweiligen hochbrechenden Schicht jeweils 0 - 5 Gew.%, vorzugsweise 0,2 - 3 Gew.% und insbesondere 0,1 - 1,5 Gew.% bezogen auf das Gesamtpigment.

Als "hochbrechende Schicht" werden in dieser Patentanmeldung die Schichten (B), (C), (C0) und (D) bezeichnet.

Vorzugsweise ist nur die Schicht (D) mit einer Erdalkalimetallverbindung oder Aluminiumverbindung dotiert.

Besonders bevorzugte Interferenzpigmente werden nachfolgend genannt:
Glimmerplättchen (natürlich) + SiO₂ + SnO₂ + TiO₂ + Fe₂O₃
Glimmerplättchen (natürlich) + Al₂O₃ + SnO₂ + TiO₂ + Fe₂O₃
Glimmerplättchen (natürlich) + SiO₂ + Al₂O₃ + SnO₂ + TiO₂ + Fe₂O₃
Glimmerplättchen (synthetisch) + SiO₂ + SnO₂ + TiO₂ + Fe₂O₃
Glimmerplättchen (synthetisch) + Al₂O₃ + SnO₂ + TiO₂ + Fe₂O₃
Glimmerplättchen (synthetisch) + SiO₂ + Al₂O₃ + SnO₂ + TiO₂ + Fe₂O₃
Glimmerplättchen (natürlich) + SiO₂ + SnO₂ + TiO₂ + TiO₂/Fe₂O₃ + Fe₂O₃
Glimmerplättchen (natürlich) + Al₂O₃ + SnO₂ + TiO₂ + TiO₂/Fe₂O₃ + Fe₂O₃
Glimmerplättchen (natürlich) + SiO₂ + Al₂O₃ + SnO₂ + TiO₂ + TiO₂/Fe₂O₃ + Fe₂O₃
Glimmerplättchen (synthetisch) + SiO₂ + SnO₂ + TiO₂ + TiO₂/Fe₂O₃ + Fe₂O₃
Glimmerplättchen (synthetisch) + Al₂O₃ + SnO₂ + TiO₂ + TiO₂/Fe₂O₃ + Fe₂O₃
Glimmerplättchen (synthetisch) + SiO₂ + Al₂O₃ + SnO₂ + TiO₂ + TiO₂/Fe₂O₃ + Fe₂O₃
Glasplättchen + SiO₂ + SnO₂ + TiO₂ + Fe₂O₃
Glasplättchen + Al₂O₃ + SnO₂ + TiO₂ + Fe₂O₃
Glasplättchen + SiO₂ + Al₂O₃ + SnO₂ + TiO₂ + Fe₂O₃
Glasplättchen + SiO₂ + SnO₂ + TiO₂ + TiO₂/Fe₂O₃ + Fe₂O₃
Glasplättchen + Al₂O₃ + SnO₂ + TiO₂ + TiO₂/Fe₂O₃ + Fe₂O₃
Glasplättchen + SiO₂ + Al₂O₃ + SnO₂ + TiO₂ + TiO₂/Fe₂O₃ + Fe₂O₃
Al₂O₃-Plättchen + SiO₂ + SnO₂ + TiO₂ + Fe₂O₃
Al₂O₃-Plättchen + Al₂O₃ + SnO₂ + TiO₂ + Fe₂O₃
Al₂O₃-Plättchen + SiO₂ + Al₂O₃ + SnO₂ + TiO₂ + Fe₂O₃
Al₂O₃-Plättchen + SiO₂ + SnO₂ + TiO₂ + TiO₂/Fe₂O₃ + Fe₂O₃
Al₂O₃-Plättchen + Al₂O₃ + SnO₂ + TiO₂ + TiO₂/Fe₂O₃ + Fe₂O₃
Al₂O₃-Plättchen + SiO₂ + Al₂O₃ + SnO₂ + TiO₂ + TiO₂/Fe₂O₃ + Fe₂O₃.

Bei den oben und nachfolgend genannten bevorzugten Interferenzpigmenten ist die Fe₂O₃-Schicht (Schicht (D)) vorzugsweise mit einer Erdalkaliverbindung, insbesondere CaO und/oder Al₂O₃, dotiert.

Sofern die erfindungsgemäßen Pigmente eine finale Schicht (E) aufweisen, sind die folgenden Mehrschichtpigmente bevorzugt:
Glimmerplättchen (natürlich) + SiO₂ + SnO₂ + TiO₂ + Fe₂O₃ + SiO₂
Glimmerplättchen (natürlich) + Al₂O₃ + SnO₂ + TiO₂ + Fe₂O₃ + SiO₂
Glimmerplättchen (natürlich) + SiO₂ + Al₂O₃ + SnO₂ + TiO₂ + Fe₂O₃ + SiO₂
Glimmerplättchen (synthetisch) + SiO₂ + SnO₂ + TiO₂ + Fe₂O₃ + SiO₂
Glimmerplättchen (synthetisch) + Al₂O₃ + SnO₂ + TiO₂ + Fe₂O₃ + SiO₂
Glimmerplättchen (synthetisch) + SiO₂ + Al₂O₃ + SnO₂ + TiO₂ + Fe₂O₃ + SiO₂
Glimmerplättchen (natürlich) + SiO₂ + SnO₂ + TiO₂ + TiO₂/Fe₂O₃ + Fe₂O₃ + SiO₂
Glimmerplättchen (natürlich) + Al₂O₃ + SnO₂ + TiO₂ + TiO₂/Fe₂O₃ + Fe₂O₃+ SiO₂
Glimmerplättchen (natürlich) + SiO₂ + Al₂O₃ + SnO₂ + TiO₂ + TiO₂/Fe₂O₃ + Fe₂O₃ + SiO₂
Glimmerplättchen (synthetisch) + SiO₂ + SnO₂ + TiO₂ + TiO₂/Fe₂O₃ + Fe₂O₃ + SiO₂
Glimmerplättchen (synthetisch) + Al₂O₃ + SnO₂ + TiO₂ + TiO₂/Fe₂O₃ + Fe₂O₃+ SiO₂
Glimmerplättchen (synthetisch) + SiO₂ + Al₂O₃ + SnO₂ + TiO₂ + TiO₂/Fe₂O₃ + Fe₂O₃+ SiO₂
Glasplättchen + SiO₂ + SnO₂ + TiO₂ + Fe₂O₃ + SiO₂
Glasplättchen + Al₂O₃ + SnO₂ + TiO₂ + Fe₂O₃ + SiO₂
Glasplättchen + SiO₂ + Al₂O₃ + SnO₂ + TiO₂ + Fe₂O₃+ SiO₂
Glasplättchen + SiO₂ + SnO₂ + TiO₂ + TiO₂/Fe₂O₃ + Fe₂O₃+ SiO₂
Glasplättchen + Al₂O₃ + SnO₂ + TiO₂ + TiO₂/Fe₂O₃ + Fe₂O₃ + SiO₂
Glasplättchen + SiO₂ + Al₂O₃ + SnO₂ + TiO₂ + TiO₂/Fe₂O₃ + Fe₂O₃ + SiO₂
Al₂O₃-Plättchen + SiO₂ + SnO₂ + TiO₂ + Fe₂O₃ + SiO₂
Al₂O₃-Plättchen + Al₂O₃ + SnO₂ + TiO₂ + Fe₂O₃ + SiO₂
Al₂O₃-Plättchen + SiO₂ + Al₂O₃ + SnO₂ + TiO₂ + Fe₂O₃ + SiO₂
Al₂O₃-Plättchen + SiO₂ + SnO₂ + TiO₂ + TiO₂/Fe₂O₃ + Fe₂O₃ + SiO₂
Al₂O₃-Plättchen + Al₂O₃ + SnO₂ + TiO₂ + TiO₂/Fe₂O₃ + Fe₂O₃ + SiO₂
Al₂O₃-Plättchen + SiO₂ + Al₂O₃ + SnO₂ + TiO₂ + TiO₂/Fe₂O₃ + Fe₂O₃ + SiO₂.

Unter den bevorzugten Mehrschichtpigmenten sind die Pigmente basierend auf Glasplättchen und synthetischen Glimmerplättchen, ferner auf Al₂O₃-Plättchen, besonders bevorzugt.

Unter hochbrechenden Beschichtungen sind die Schichten mit einem Brechungsindex von ≥ 1,8, unter niedrigbrechenden Schichten solche mit n < 1,8 in dieser Anmeldung zu verstehen.

Die erfindungsgemäßen Mehrschichtpigmente lassen sich in der Regel relativ leicht herstellen.

Die Metalloxidschichten werden vorzugsweise nasschemisch aufgebracht, wobei die zur Herstellung von Perlglanzpigmenten entwickelten nasschemischen Beschichtungsverfahren angewendet werden können. Derartige Verfahren sind z.B. beschrieben in DE 14 67 468, DE 19 59 988, DE 20 09 566, DE 22 14 545, DE 22 15 191, DE 22 44, 298, DE 23 13 331, DE 15 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen.

Bei der Nassbeschichtung werden die Substratplättchen in Wasser suspendiert und mit ein oder mehreren hydrolysierbaren Metallsalzen oder einer Wasserglaslösung bei einem für die Hydrolyse geeigneten pH-Wert versetzt, der so gewählt wird, dass die Metalloxide bzw. Metalloxidhydrate direkt auf den Plättchen ausgefällt werden, ohne dass es zu Nebenfällungen kommt. Der pH-Wert wird üblicherweise durch gleichzeitiges Zudosieren einer Base und/oder Säure konstant gehalten. Anschließend werden die Pigmente abgetrennt, gewaschen und bei 50-150 °C für 6-18 h getrocknet und gegebenenfalls 0,5-3 h geglüht, wobei die Glühtemperatur im Hinblick auf die jeweils vorliegende Beschichtung optimiert werden kann. In der Regel liegen die Glühtemperaturen zwischen 600 und 1000 °C, vorzugsweise zwischen 600 und 900 °C. Falls gewünscht, können die Pigmente nach Aufbringen einzelner Beschichtungen abgetrennt, getrocknet und ggf. geglüht werden, um dann zur Auffällung der weiteren Schichten wieder resuspendiert zu werden.

Die Auffällung der SiO₂-Schicht auf das Substrat erfolgt in der Regel durch Zugabe einer Kalium- oder Natronwasserglas-Lösung bei einem geeigneten pH-Wert.

Weiterhin kann die Beschichtung auch in einem Wirbelbettreaktor durch Gasphasenbeschichtung erfolgen, wobei z. B. die in EP 0 045 851 und EP 0 106 235 zur Herstellung von Perlglanzpigmenten vorgeschlagenen Verfahren entsprechend angewendet werden können.

Der Farbton der Mehrschichtpigmente kann in sehr weiten Grenzen durch die unterschiedliche Wahl der Belegungsmengen bzw. der daraus resultierenden Schichtdicken variiert werden. Die Feinabstimmung für einen bestimmten Farbton kann über die reine Mengenwahl hinaus durch visuell oder messtechnisch kontrolliertes Anfahren der gewünschten Farbe erreicht werden.

Zur Erhöhung der Licht-, Wasser- und Wetterstabilität empfiehlt es sich häufig, in Abhängigkeit vom Einsatzgebiet, das fertige Pigment einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung (Schicht E) wird die chemische und photochemische Stabilität weiter erhöht oder die Handhabung des Pigments, insbesondere die Einarbeitung in unterschiedliche Medien, erleichtert. Zur Verbesserung der Benetzbarkeit, Dispergierbarkeit und/oder Verträglichkeit mit den Anwendermedien können beispielsweise funktionelle Beschichtungen aus Al₂O₃ oder ZrO₂ oder deren Gemische auf die Pigmentoberfläche aufgebracht werden. Weiterhin sind organische Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjeé, Philips Technical Review, Vol. 44, No. 3, 81 ff. und P.H. Harding J.C. Berg, J. Adhesion Sci. Technol. Vol. 11 No. 4, S. 471-493. Die Schicht (E) weist vorzugsweise Dicken von 0,1 - 100 nm, insbesondere von 0,1 - 50 nm und ganz besonders bevorzugt von 0,1 - 30 nm auf.

In einer bevorzugten Ausführungsform besteht die Schicht (E) aus einer SiO₂-Schicht. Diese Schicht kann sowohl kalziniert als auch nicht kalziniert sein. Vorzugsweise handelt es sich um eine kalzinierte SiO₂-Schicht.

In einer weiteren bevorzugten Ausführungsform besteht die äußere optionale Schutzschicht (E) aus einer oder zwei Metalloxidschichten der Elemente Si, Al, Zn oder Ce. Insbesondere bevorzugt ist hierbei eine Schichtenfolge bei der zunächst eine Ceroxidschicht aufgebracht ist, der dann eine SiO₂-Schicht folgt, wie beispielsweise in der WO 2006/021386 A1 beschrieben.

Die äußere Schutzschicht kann des Weiteren an der Oberfläche organisch-chemisch modifiziert sein. Beispielsweise können ein oder mehrere Silane auf dieser äußeren Schutzschicht aufgebracht sein. Bei den Silanen kann es sich um Alkylsilane mit verzweigten oder unverzweigten Alkylresten mit 1 bis 24 C-Atomen, vorzugsweise 6 bis 18 C-Atomen handeln.

Bei den Silanen kann es sich aber auch um organofunktionelle Silane handeln, die eine chemische Anbindung an einen Kunststoff, ein Bindemittel eines Lackes oder einer Farbe, etc. ermöglichen.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Degussa, Rheinfelden, Deutschland, hergestellt und unter dem Handelsnamen "Dynasylan®" vertrieben. Weitere Produkte können von der Fa. OSi Specialties (Silquest®-Silane) oder von der Fa. Wacker, beispielsweise Standard- und α-Silane aus der GENIOSIL®-Produktgruppe, bezogen werden.

Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), 3-Mercaptopropyltri(m)ethoxysilan (Dynasylan MTMO oder 3201; Silquest A-189), 3-Glycidoxypropyltrimethoxysilan (Dynasylan GLYMO, Silquest A-187), tris-(3-Trimethoxysilylpropyl)isocyanurat (Silquest Y-11597), gamma-Mercaptopropyltrimethoxysilan (Silquest A-189), Bis-(3-Triethoxysilylpropyl)polysulfid (Silquest A-1289), Bis-(3-Triethoxysilyl)disulfid (Silquest A-1589), beta-(3,4-Epoxycyclohexyl)ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), (Methacryloxymethyl)tri(m)ethoxysilan (GENIOSIL XL 33, XL 36), (Methacryloxymethyl)(m)ethyldimethoxysilan (GENIOSIL XL 32, XL 34), Isocyanatomethyl)trimethoxysilan (GENIOSIL XL 43), (Isocyanatomethyl)methyldimethoxysilan (GENIOSIL XL 42), (Isocyanatomethyl)trimethoxysilan (GENIOSIL XL 43) 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltriacetoxysilan, 3-Methacryloxypropymethytdimethoxysilan, Vinyltrichlorsilan, Vinyltrimethoxysilan (GENIOSIL XL 10), Vinyltris(2-methoxyethoxy)silan (GENIOSIL GF 58), Vinyltriacetoxysilan.

Es ist aber auch möglich, andere organofunktionelle Silane auf den erfindungsgemäßen Effektpigmenten zu verwenden.

Weiterhin lassen sich, beispielsweise kommerziell von Degussa erhältliche, wässrige Vorhydrolysate einsetzen. Hierzu gehören u.a. wässriges, alkoholfreies Aminosilanhydrolysat (Dynasylan Hydrosil 1151), wässriges, alkoholfreies amino/alkylfunktionelles Siloxancooligomer (Dynasylan Hydrosil 2627), wässriges, alkoholfreies diaminolalkylfunktionelles Siloxancooligomer (Dynasylan Hydrosil 2776), wässriges, alkoholfreies amino/vinylfunktionelles Siloxancooligomer (Dynasylan Hydrosil 2907), wässriges, alkoholfreies amino/alkylfunktionelles Siloxancooligomer (Dynasylan Hydrosil 2909), wässriges, alkoholfreies epoxyfunktionelles Siloxanoligomer (Dynasylan Hydrosil 2926) oder wässriges, alkoholfreies amino/methacrylatfunktionelles Siloxancooligomer (Dynasylan Hydrosil 2929), oligomeres Diaminosilansystem (Dynasylan 1146), vinyl/alkylfunktionelles Siloxancooligomer (Dynasylan 6598), vinyl- und methoxygruppenhaltiges Vinylsilankonzentrat (oligomeres Siloxan) (Dynasylan 6490) oder oligomeres kurzkettiges alkylfunktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Effektpigmentes an verschiedenartige Bindemittel sehr gut geeignet.

Bevorzugt werden hierzu folgende Verbindungen genommen:
Aminopropyltrimethoxysilan (Dynasylan AMMO; Silquest A-1110), Aminopropyltriethoxysilan (Dynasylan AMEO) oder N-(2-Aminoethyl)-3-aminopropyltrimethoxysilan (Dynasylan DAMO, Silquest A-1120) oder N-(2-Aminoethyl)-3-aminopropyltriethoxysilan, Triaminofunktionelles Trimethoxysilan (Silquest A-1130), bis-(gamma-Trimethoxysilylpropyl)amin (Silquest A-1170), N-ethyl-gammaaminoisobutyltrimethoxysilan (Silquest A-Link 15), N-Phenylgammaaminopropyltrimethoxysilan (Silquest Y-9669), 4-Amino-3,3-dimethylbutyltrimethoxysilan (Silquest Y-11637), N-Cyclohexylaminomethylmethyldiethoxysilan (GENIOSIL XL 924), (N-Cyclohexylaminomethyl)triethoxysilan (GENIOSIL XL 926), (N-Phenylaminomethyl)trimethoxysilan (GENIOSIL XL 973) und deren Mischungen.

Bei einer weiterhin bevorzugten Ausführungsform ist das Silan ohne funktionelle Bindungsgruppe ein Alkylsilan. Das Alkylsilan weist vorzugsweise die Formel (A) auf:

R_{(4-z)}Si(X)_{Z} (A)

Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

Ein derartiges Silan bewirkt eine stärkere Hydrophobierung der Pigmentoberfläche. Diese wiederum führt dazu, dass das derart beschichtete Perlglanzpigment in der Lackbeschichtung tendenziell nach oben aufschwimmt. Bei plättchenförmigen Effektpigmenten wird ein derartiges Verhalten als "leafing"-Verhalten bezeichnet.

Eine Silanmischung bestehend aus mindestens einem Silan, welches wenigstens eine funktionelle Gruppe besitzt, die eine Anbindung an das Bindemittel ermöglicht, und einem, in Wasser unlöslichen oder kaum löslichen, Alkylsilan ohne Aminogruppe,führt in der Regel zu optimalen anwendungstechnischen Eigenschaften der Perlglanzpigmente. Eine solche organisch-chemische Oberflächenmodifizierung führt dazu, dass sich die Effektpigmente in einer Lack- oder Farbschicht ausgezeichnet orientieren, d.h. im Wesentlichen planparallel zu dem lackierten bzw. angestrichenen Untergrund, und zugleich chemisch mit dem Bindemittelsystem des Lackes bzw. der Farbe reagieren und mithin kovalent in der Lack- bzw. Farbschicht gebunden sind. Derartige Lack- bzw. Farbschichten weisen eine erhöhte mechanische und chemische Beständigkeit gegenüber Umwelteinflüssen, wie z.B. Wetter, etc., auf.

Da die erfindungsgemäßen Mehrschichtpigmente einen starken Glanz mit intensiven Interferenzfarben und einer ansprechenden Pulverfarbe verbinden, lassen sich mit ihnen besonders wirksame Effekte in den verschiedenen Anwendungsmedien erzielen, z. B. in kosmetischen Formulierungen, wie z. B. Nagellacken, Lippenstiften, Presspudern, Gelen, Lotionen, Seifen, Zahnpasten, in Lacken wie z.B. Autolacken, Industrielacken und Pulverlacken sowie in Druckfarben, Saatguteinfärbungen, Kunststoffen und in der Keramik.

Die Konzentration des erfindungsgemäßen Pigments im zu pigmentierenden Anwendungssystem liegt in der Regel zwischen 0,1 und 100 Gew.%, vorzugsweise zwischen 0,1 und 70 Gew.% und insbesondere zwischen 0,5 und 10 Gew.%, bezogen auf den Gesamtfestkörpergehalt des Systems. Sie ist in der Regel abhängig vom konkreten Anwendungsfall.

Es versteht sich von selbst, dass für die verschiedenen Anwendungszwecke die erfindungsgemäßen Mehrschichtpigmente auch vorteilhaft in Abmischung mit einem oder mehreren Farbmitteln, z.B. Effektpigmenten ausgewählt aus der Gruppe der Perlglanzpigmente, Interferenzpigmente, goniochromatischen Pigmente, BiOCI-Plättchen, Mehrschichtpigmente, Metallpigmente, Glanzpigmenten, und/oder organischen Farbstoffen, und/oder organischen Farbpigmenten und anderen Pigmenten, wie z.B. transparenten und deckenden Weiß-, Bunt- und Schwarzpigmenten sowie mit plättchenförmigen Eisenoxiden, holographischen Pigmenten, LCPs (Liquid Crystal Polymers) und herkömmlichen transparenten, bunten und schwarzen Glanzpigmenten auf der Basis von metalloxidbeschichteten Glimmer- und SiO₂-Plättchen, etc., verwendet werden können. Die erfindungsgemäßen Mehrschichtpigmente können in jedem Verhältnis mit einem Farbmittel gemischt werden. Das Gewichtsverhältnis Mehrschichtpigment zu Farbmittel kann je nach Farbausprägung 1 : 99 zu 99 : 1 sein.

Als Farbmittel kommen vor allem Perlglanzpigmente insbesondere auf Basis von natürlichem oder synthetischem Glimmer, SiO₂-Plättchen, Fe₂O₃-Plättchen, Glasplättchen oder Al₂O₃-Plättchen, die mit einer oder mehreren Metalloxidschichten umhüllt sind, Metalleffektpigmente (Al-Plättchen, Bronzen), optisch variable Pigmente (OVP's), Flüssigkristallpolymerpigmente (LCP's) oder holographische Pigmente in Frage.

Zu den sphärischen Farbmitteln zählen insbesondere TiO₂, eingefärbtes SiO₂, CaSO₄, Eisenoxide, Chromoxide, Ruß, organische Farbpigmente, wie z.B. Anthrachinon-Pigmente, Chinacridon-Pigmente, Diketopyrrolopyrrol-Pigmente, Phthalocyanin-Pigmente, Azopigmente, Isoindolin-Pigmente. Bei den nadelförmigen Pigmenten handelt es sich vorzugsweise um BiOCl, eingefärbte Glasfasern, α-FeOOH, organische Farbpigmente, wie z.B. Azopigmente, β-Phthalocyanin Cl Blue 15,3, Cromophtalgelb 8GN (Ciba-Geigy), Irgalith Blau PD56 (Ciba-Geigy), Azomethinkupferkomplex Cl Yellow 129, Irgazingelb 5GT (Ciba-Geigy).

Geeignete organische Farbpigmente und Farbstoffe sind natürlichen oder synthetischen Ursprungs, wie z.B. Chromoxid, Ultramarin.

Die erfindungsgemäßen Mehrschichtpigmente können selbstverständlich auch mit Füllstoffen in jedem Gewichtsverhältnis miteinander abgemischt werden bzw. eingesetzt werden. Als Füllstoffe sind z. B. zu nennen synthetische organische Polymere, Polymethylmethacrylat, Methylmethacrylat Cross-Polymer, natürlicher und synthetischer Glimmer, Nylon-Pulver, reine oder gefüllte Melaminharze, Talkum, SiO₂, BN, Glaspulver, Glaskugeln, Kaolin, Oxide oder Hydroxide von Aluminium, Magnesium, Calcium, Zink, BiOCl, Bariumsulfat, Calciumsulfat, Calciumcarbonat, Magnesiumcarbonat, basische Erdalkalicarbonate, wie z.B. Calcium- oder Magnesiumcarbonat, Kohlenstoff, sowie physikalische oder chemische Kombinationen dieser Stoffe. Bezüglich der Partikelform der Füllstoffe gibt es keine Einschränkungen. Sie kann den Anforderungen gemäß z. B. unregelmäßig, plättchenförmig, sphärisch oder nadelförmig sein.

Ebenso können nanoskalige Dielektrika, insbesondere in kosmetischen Formulierungen zur Verbesserung des Hautgefühls, den Mehrschichtpigmenten beigemischt werden. Beispiele für derartige Beimischungen sind Al₂O₃, SiO₂, ZnO oder TiO₂, die üblicherweise in Mengen von 0,01 - 15 Gew.% der Formulierung zugegeben werden.

Die erfindungsgemäßen Mehrschichtpigmente sind mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die erfindungsgemäßen Mehrschichtpigmente auch für die Lasermarkierung von Papier und Kunststoffen, sowie für Anwendungen im Agrarbereich, z. B. für Gewächshausfolien, sowie z. B. für die Farbgebung von Zeltplanen, geeignet.

Bei der Pigmentierung von Bindemittelsystemen z. B. für Farben und Druckfarben für den Tiefdruck, Offsetdruck oder Siebdruck, oder als Vorprodukt für Druckfarben, hat sich der Einsatz der erfindungsgemäßen Mehrschichtpigmente in Form von hochpigmentierten Pasten, Granulaten, Pellets, etc., als besonders geeignet erwiesen. Das erfindungsgemäße Pigment wird in der Regel in die Druckfarbe in Mengen von 2-35 Gew.%, vorzugsweise 5-25 Gew.%, und insbesondere 8-20 Gew.% eingearbeitet. Offsetdruckfarben können die Pigmente bis zu 40 Gew.% und mehr enthalten. Die Vorprodukte für die Druckfarben, z.B. in Granulatform, als Pellets, Briketts, etc., enthalten neben dem Bindemittel und Additiven bis zu 98 Gew.% des erfindungsgemäßen Pigments. Die Druckfarben enthaltend das erfindungsgemäße Pigment zeigen in der Regel reinere Farbtöne als mit herkömmlichen Effektpigmenten.

Die erfindungsgemäßen Mehrschichtpigmente sind weiterhin geeignet zur Herstellung von fließfähigen Pigmentpräparationen und Trockenpräparaten, insbesondere für Druckfarben, enthaltend ein oder mehrere erfindungsgemäße Pigmente, Bindemittel und optional ein oder mehrere Additive.

In Kunststoffen enthaltend das erfindungsgemäße Mehrschichtpigment, vorzugsweise in Mengen von 0,01 bis 50 Gew.%, insbesondere 0,1 bis 7 Gew.%, lassen sich besonders ausgeprägte Farbeffekte erzielen.

Im Lackbereich, insbesondere im Automobillack, wird das Mehrschichtpigment, auch für 3-Schichtaufbauten in Mengen von 0,1-20 Gew.%, vorzugsweise 1 bis 10 Gew.%, eingesetzt.

Weiterhin kann das erfindungsgemäße Pigment zur Veredlung von Lebensmitteln, z. B. Masse-Einfärbung und/oder Überzüge von Bonbons, Weingummi, wie z.B. Gummibärchen, Pralinen, Lakritze, Konfekt, Zuckerstangen, Puddings, Brausegetränke, Limonaden, etc., oder als Überzug, z.B. bei Dragees und Tabletten im Pharmabereich, eingesetzt werden.

Das erfindungsgemäße Mehrschichtpigment kann auch vorteilhaft in der dekorativen und pflegenden Kosmetik eingesetzt werden. Die Einsatzkonzentration reicht von 0,01 Gew.% im Shampoo bis zu 100 Gew. % bei losen Pudern. Bei einer Mischung der erfindungsgemäßen Pigmente mit Füllstoffen, vorzugsweise mit sphärischen Füllstoffen, wie z. B. SiO₂, kann die Konzentration bei 0,01-70 Gew.% in der kosmetischen Formulierung liegen. Die kosmetischen Produkte, wie z.B. Nagellacke, Presspuder, Shampoos, lose Puder, Lippenstifte und Gele, zeichnen sich durch besonders interessante Farbeffekte, Sparkle und einen hohen Glanz aus. Den Konzentrationen der erfindungsgemäßen Mehrschichtpigmente in der Formulierung sind keine Grenzen gesetzt. Sie können - je nach Anwendungsfall - zwischen 0,001 (rinse-off-Produkte, z. B. Duschgele) und 100 % (z. B. Glanzeffekt-Artikel für besondere Anwendungen) liegen.

Aufgrund des guten Skin Feelings und der sehr guten Hautadhäsion sind die erfindungsgemäßen Pigmente sowohl für Personal Care Applications, wie z. B. Body Lotions, Emulsionen, Shampoos, Seifen, etc., als auch insbesondere für die dekorative Kosmetik geeignet.

Selbstverständlich können die erfindungsgemäßen Mehrschichtpigmente in den Formulierungen auch mit jeder Art von Roh- und Hilfsstoffen sowie Wirkstoffen kombiniert werden. Dazu gehören u.a. Wasser, Alkohole, Polyole, polare und unpolare Öle, Fette, Wachse, Filmbildner, Polymere, Co-Polymere, Tenside, Radikalfänger, Antioxidantien, wie z.B. Vitamin C oder Vitamin E, Stabilisatoren, Geruchsverstärker, Silikonöle, Emulgatoren, Duftstoffe, Lösemittel wie z.B. Ethanol, Ethylacetat oder Butylacetat, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliciumdioxide, Ca-Silicate, Gelatine, hochmolekulare Kohlenhydrate und/oder oberflächenaktive Hilfsmittel, etc.

Geeignete Wirkstoffe sind z.B. Insect Repellents, anorganische UV-Filter, wie z.B. TiO₂, UV A/BC-Schutzfilter (z.B. OMC, B3, MBC), auch in verkapselter Form, Anti-Ageing-Wirkstoffe, Vitamine und deren Derivate (z.B. Vitamin A, C, E, etc.), Selbstbräuner (z.B. DHA, Erytrolyse, u.a.) sowie weitere kosmetische Wirkstoffe, wie z.B. Bisabolol, LPO, VTA, Ectoin, Emblica, Allantoin, Bioflavanoide und deren Derivate.

Organische UV-Filter werden in der Regel in einer Menge von 0,5 bis 10 Gew.%, vorzugsweise 1 bis 8 Gew.%, anorganische Filter von 0,1 bis 30 Gew.% in kosmetische Formulierungen eingearbeitet.

Die erfindungsgemäßen Zubereitungen können darüber hinaus weitere übliche hautschonende oder hautpflegende Wirkstoffe enthalten. Dies können prinzipiell alle dem Fachmann bekannten Wirkstoffe sein. Besonders bevorzugte Wirkstoffe sind Pyrimidincarbonsäuren und/oder Aryloxime.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Up, Pflegecremes und Sonnenschutzpräparaten eingesetzt werden.

Bei Selbstbräunungscremes, -lotionen, -sprays, etc. enthaltend beispielsweise den Selbstbräuner DHA (Dihydroxyaceton) und ein Effektpigment mit abschließender TiO₂-Schicht, z.B. ein mit TiO₂ (Anatas) beschichtetes Glasplättchen, wird das DHA langsam in der Formulierung abgebaut. Kosmetische Formulierungen enthaltend DHA und das erfindungsgemäße Pigment, insbesondere ein Pigment mit einer finalen Schicht (E) aus SiO₂, zeichnen sich dadurch aus, dass das DHA in seiner Wirkung voll erhalten bleibt.

Die die erfindungsgemäßen Mehrschichtpigmente enthaltenden Formulierungen können dem lipophilen, hydrophilen oder hydrophoben Typ angehören. Bei heterogenen Formulierungen mit diskreten wässrigen und nicht-wässrigen Phasen können die erfindungsgemäßen Pigmente in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Als Anwendungsform der kosmetischen Formulierungen seien z.B. genannt Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können neben den erfindungsgemäßen Pigmenten beliebige übliche Trägerstoffe, Hilfsstoffe und gegebenenfalls weitere Wirkstoffe zugesetzt werden.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie z.B. Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Die kosmetischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-ÖI-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Weitere Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Feste Stifte bestehen aus natürlichen oder synthetischen Wachsen und Ölen, Fettalkoholen, Fettsäuren, Fettsäureestern, Lanolin und anderen Fettkörpern.

Bei den kosmetischen Ölen handelt es sich vorzugsweise um Mineralöl, hydriertes Polyisobuten, synthetisches oder aus Naturprodukten hergestelltes Squalan, kosmetische Ester oder Ether, die verzweigt oder unverzweigt, gesättigt oder ungesättigt sein können, pflanzliche Öle oder Gemische davon.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen Mehrschichtpigmente können beispielsweise in Lippenstiften, Lipgloss, Rouge, Eyeliner, Lidschatten, (Volumen)Mascara, Nagellacke, Tagescremes, Nachtcremes, Körperlotionen, Reinigungsmilch, Körperpuder, Haargele, Haarmasken, Haarspülungen, Haarshampoos, Duschgelen, Duschölen, Badeölen, Sonnenschutz, Prä-Sun- und After-Sun-Präparate, Bräunungslotionen, Bräunungssprays, Make-ups, Lotions, Seifen, Badesalze, Zahnpasta, Gesichtsmasken, Presspuder, lose Puder und Gele, etc., verwendet werden. Die Herstellung derartiger Produkte erfolgt auf eine Weise, wie sie dem Fachmann auf diesem Gebiet bekannt ist.

Gegenstand der Erfindung sind insbesondere Formulierungen, die neben dem erfindungsgemäßen Mehrschichtpigment mindestens einen Bestandteil ausgewählt aus der Gruppe der Absorptionsmittel, Adstringenzien, antimikrobiellen Stoffen, Antioxidantien, Antiperspirantien, Antischaum-mitteln, Antischuppenwirkstoffen, Antistatika, Bindemitteln, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

Gegenstand der Erfindung ist weiterhin auch die Verwendung der Pigmente in Formulierungen wie Farben, Lacken, Automobillacken, Pulverlacken, Druckfarben, Sicherheitsdruckfarben, Kunststoffen, keramischen Materialien, Gläsern, Papier, in Tonern für elektrophotographische Druckverfahren, im Saatgut, in Gewächshausfolien und Zeltplanen, als Absorber bei der Lasermarkierung von Papier und Kunststoffen, in kosmetischen Formulierungen, zur Herstellung von Pigmentanteigungen mit Wasser, organischen und/oder wässrigen Lösemitteln, zur Herstellung von Pigmentpräparationen und Trockenpräparaten, wie z. B. Granulate, zur Masseeinfärbung von Lebensmitteln, zur Einfärbung von Überzügen von Lebensmittelprodukten und Pharmaerzeugnissen, z.B. als Überzug bei Dragees und Tabletten, in Wertdokumenten, wie z.B. Banknoten, Schecks, Scheckkarten, etc.

Die nachfolgenden Beispiele sollen die Erfindung näher erläutern ohne sie jedoch zu beschränken.

### Beispiele

Sofern als Substrat Glasplättchen eingesetzt werden, ist die chemische Zusammensetzung der Glasplättchen aufgrund der Belegung mit einer SiO₂-Schicht (Schicht (A0)) von untergeordneter Bedeutung für die weiteren Beschichtungen und die resultierenden anwendungstechnischen Eigenschaften der finalen Pigmente. Geeignete Glaszusammensetzungen sind z. B. solche, wie in den Tabellen 1 und 2 angegeben.

**Tabelle 1: Glaszusammensetzungen in %**

| Bestandteile | Glas A | Glas B |
|---|---|---|
| SiO₂ | 64 | 60 |
| Al₂O₃ | 5 | 5 |
| CaO | 6,2 | 7,7 |
| MgO | 2,2 | 5,2 |
| B₂O₃ | 5,3 | 6,1 |
| Na₂O + K₂O | 13,5 | 16 |
| ZnO | 3,7 | 0 |
| FeO/Fe₂O₃ | 0,1 | 0 |

**Tabelle 2: Glaszusammensetzungen in %**

| Bestandteile | Glas C | Glas D |
|---|---|---|
| SiO₂ | 65,7 | 64,8 |
| Al₂O₃ | 4,0 | 4,9 |
| CaO | 5,9 | 5,6 |
| MgO | 1,9 | 1,7 |
| B₂O₃ | 5,4 | 4,2 |
| Na₂O + K₂O | 12,7 | 14,7 |
| ZnO | 4,3 | 3,9 |
| FeO/Fe₂O₃ | 0,1 | 0,2 |

### Beispiel 1

200 g Glasplättchen (mit einer Zusammensetzung wie in oben stehender Tabelle 1 unter Glas A beschrieben, einer mittleren Flakedicke von 850 nm und einem D₅₀-Wert laut Malvern Mastersizer 2000 von ca. 80 µm; Hersteller: Merck KGaA) werden in 2000 ml VE-Wasser suspendiert und unter Rühren auf 70 °C erhitzt. Der pH-Wert wird auf 9,0 eingestellt. Es werden nun innerhalb einer Stunde 50 g Natronwasserglas-Lösung (W_{(SiO2)} = 0,2) zudosiert. Hierbei wird der pH-Wert durch Zugabe von Salzsäure konstant bei 9,0 gehalten. Der pH-Wert wird nun mit Salzsäure auf 2,0 eingestellt. Nachfolgend werden 28 g einer AlCl₃-Lösung (w_{(AlCl3)} = 0,29) innerhalb 10 Minuten zugegeben Als nächstes erfolgt innerhalb einer Stunde die Zugabe von 150 ml einer SnCl₄-Lösung (w_{(SnCl4)} = 0,02) und (w_{(HCl)} = 0,04). Der pH-Wert wird mit Natronlauge konstant bei 2,0 gehalten. Nach Beendigung der Dosierung der SnCl₄-Lösung wird der pH-Wert mit Salzsäure auf 1,6 eingestellt, und die Temperatur der Suspension wird auf 85 °C erhöht. Nun beginnt die Zugabe von ca. 200 ml TiOCl₂-Lösung (w_{(TiCl4)} = 400 g/l). Die Dosierung wird beim Erreichen des gewünschten koloristischen Endpunkts unterbrochen. Der pH-Wert wird hierbei mit Natronlauge konstant gehalten und anschließend auf 3,2 eingestellt. Im nächsten Schritt werden innerhalb von 60 Minuten 100 g einer FeCl₃-Lösung (w_{(Fe)} = 0,10) zugesetzt, dann wird weiterhin soviel FeCl₃-Lösung hinzugefügt bis der gewünschte Farbton erreicht wird. Der pH-Wert wird hierbei mit NaOH konstant gehalten und nach Ende der Dosierung der FeCl₃-Lösung wird der pH auf 7,0 eingestellt. Die Pigmentsuspension wird über eine Saugnutsche filtriert, und mit VE-Wasser werden Salzrückstände ausgewaschen. Es folgt die Trocknung über Nacht bei 110 °C. Anschließend wird das Pigment 30 Minuten bei 650 °C geglüht.

### Beispiel 2

200 g mit Rußpartikeln dotierte Glasplättchen (mit einer Zusammensetzung wie in oben stehender Tabelle 1 unter Glas C beschrieben und mit 0,5 % Ruß (bezogen auf das Glasplättchen) dotiert, mittlere Flakedicke 850 nm, D₅₀ = 80 µm laut Malvern Mastersizer 2000; Hersteller: Merck KGaA) werden in 2000 ml VE-Wasser suspendiert und unter Rühren auf 70 °C erhitzt. Der pH-Wert wird auf 9,0 eingestellt. Es werden nun innerhalb einer Stunde 50 g Natronwasserglas-Lösung (w_{(SiO2)} = 0,2) zudosiert. Hierbei wird der pH-Wert durch Zugabe von Salzsäure konstant bei 9,0 gehalten. Der pH-Wert wird nun mit Salzsäure auf 2,0 eingestellt. Nachfolgend werden 28 g einer AlCl₃-Lösung (w_{(AlCl3)} = 0,29) innerhalb 10 Minuten zugegeben Als nächstes erfolgt innerhalb einer Stunde die Zugabe von 150 ml einer SnCl₄-Lösung (w_{(SnCl4)} = 0,02) und (w_{(HCl)} = 0,04). Der pH-Wert wird mit Natronlauge konstant bei 2,0 gehalten. Nach Beendigung der Dosierung der SnCl₄-Lösung wird der pH-Wert mit Salzsäure auf 1,6 eingestellt, und die Temperatur der Suspension wird auf 85 °C erhöht. Nun beginnt die Zugabe von ca. 200 ml TiOCl₂-Lösung (w_{(TiCl4)} = 400 g/l). Die Dosierung wird beim Erreichen des gewünschten koloristischen Endpunkts unterbrochen. Der pH-Wert wird hierbei mit Natronlauge konstant gehalten und anschließend auf 3,2 eingestellt. Im nächsten Schritt werden innerhalb von 60 Minuten 100 g einer FeCl₃-Lösung (w_{(Fe)} = 0,10) zugesetzt, dann wird weiterhin soviel FeCl₃-Lösung hinzugefügt bis der gewünschte Farbton erreicht wird. Der pH-Wert wird hierbei mit NaOH konstant gehalten und nach Ende der Dosierung der FeCl₃-Lösung wird der pH auf 7,0 eingestellt. Die Pigmentsuspension wird über eine Saugnutsche filtriert, und mit VE-Wasser werden Salzrückstände ausgewaschen. Es folgt die Trocknung über Nacht bei 110 °C. Anschließend wird das Pigment 30 Minuten bei 650 °C geglüht.

### Beispiel 3

Analog Beispiel 1 werden 150 g synthetische Glimmerplättchen (D₅₀ = 20 µm, bestimmt mit Malvern Mastersizer 2000) in 2000 ml VE-Wasser suspendiert und unter Rühren auf 75 °C erhitzt. Der pH-Wert wird nun mit Salzsäure auf 2,0 eingestellt. Nachfolgend werden 56 g einer AlCl₃-Lösung (w_{(AlCl3)} = 0,29) innerhalb von 10 Minuten zugegeben. Als nächstes erfolgt innerhalb einer Stunde die Zugabe von 300 ml einer SnCl₄-Lösung (w(SnCl₄ = 0,02) und (w_{(HCl)}=0,04). Der pH-Wert wird mit Natronlauge konstant bei 2,0 gehalten. Nach Beendigung der Dosierung der SnCl₄-Lösung wird der pH-Wert mit Salzsäure auf 1,6 eingestellt. Nun beginnt die Zugabe von ca. 200 ml TiOCl₂-Lösung (w_{(TiCl4)}= 400 g/l). Die Dosierung wird beim Erreichen des gewünschten koloristischen Endpunkts unterbrochen. Der pH-Wert wird hierbei mit NaOH konstant gehalten und anschließend auf 3,2 eingestellt. Im nächsten Schirtt werden innerhalb von 60 Minuten 100 g einer FeCl₃-Lösung (w_{(Fe)} = 0,10) zugesetzt, dann weiterhin soviel FeCl₃-Lösung zugetropft, bis der gewünschte Farbton errreicht wird. Der pH-Wert wird hierbei mit NaOH konstant gehalten. Nach Ende der Dosierung der FeCl₃-Lösung wird der pH-Wert auf 7,0 erhöht. Die Pigmentsuspension wird über eine Saugnutsche filtriert, und mit VE-Wasser werden Salzrückstände ausgewaschen. Es folgt die Trocknung über Nacht bei 100 °C. Zuletzt wird das Pigment 30 Minuten bei 800 °C geglüht.

### Beispiel 4

Analog Beispiel 1 werden 100 g Al₂O₃-Plättchen der Teilchengröße D₅₀-Wert = 15 µm gemessen mit Malvern Mastersizer 2000) in 2000 ml VE-Wasser suspendiert und unter Rühren auf 70 °C erhitzt. Der pH-Wert wird auf 9,0 eingestellt. Es werden nun innerhalb einer Stunde 80 g Natronwasserglas-Lösung (w_{(SiO2)} = 0,2) zudosiert. Hierbei wird der pH-Wert durch Zugabe von Salzsäure konstant bei 9,0 gehalten. Der pH-Wert wird nun mit Salzsäure auf 2,0 eingestellt. Als nächstes erfolgt innerhalb einer Stunde die Zugabe von 300 ml einer SnCl₄-Lösung (w_{(SnCl4)}=0,02) und (w_{(HCl)} = 0,04). Der pH-Wert wird mit Natronlauge konstant bei 2,0 gehalten. Nach Beendigung der Dosierung der SnCl₄-Lösung wird der pH-Wert mit Salzsäure auf 1,8 eingestellt, und die Temperatur der Suspension wird auf 80 °C erhöht. Nun beginnt die Zugabe von ca. 200 ml TiOCl₂-Lösung (w_{(TiCl4)} = 400 g/l). Die Dosierung wird beim Erreichen des gewünschten koloristischen Endpunkts unterbrochen. Der pH-Wert wird hierbei mit Natronlauge konstant gehalten und anschließend auf 3,2 eingestellt. Im nächsten Schritt werden innerhalb von 60 Minuten 100 g einer FeCl₃-Lösung (w_{(Fe)}=0,10) zugesetzt, dann wird weiterhin soviel FeCl₃-Lösung hinzugefügt bis der gewünschte Farbton erreicht wird. Der pH-Wert wird hierbei mit NaOH konstant gehalten. Nach Ende der Dosierung der FeCl₃-Lösung wird der pH auf 6,0 eingestellt. Zuletzt wird die Pigmentsuspension mit einer Saugnutsche filtriert und die Salzrückstände werden ausgewaschen. Es folgt die Trocknung über Nacht bei 110 °C. Anschließend wird das Pigment 30 Minuten bei 700 °C geglüht.

### Beispiel 5

Analog Beispiel 1 werden 100 g Al₂O₃-Plättchen der Teilchengröße D₅₀-Wert = 15 µm gemessen mit Malvern Mastersizer 2000) in 2000 ml VE-Wasser suspendiert und unter Rühren auf 70 °C erhitzt. Der pH-Wert wird auf 9,0 eingestellt. Es werden nun innerhalb einer Stunde 80 g Natronwasserglas-Lösung (w_{(SiO2)} = 0,2) zudosiert. Hierbei wird der pH-Wert durch Zugabe von Salzsäure konstant bei 9,0 gehalten. Der pH-Wert wird nun mit Salzsäure auf 2,0 eingestellt. Als nächstes erfolgt innerhalb einer Stunde die Zugabe von 300 ml einer SnCl₄-Lösung (w_{(SnCl4)}=0,02) und (w_{(HCl)} = 0,04). Der pH-Wert wird mit Natronlauge konstant bei 2,0 gehalten. Nach Beendigung der Dosierung der SnCl₄-Lösung wird der pH-Wert mit Salzsäure auf 1,8 eingestellt, und die Temperatur der Suspension wird auf 80 °C erhöht. Nun beginnt die Zugabe von ca. 200 ml TiOCl₂-Lösung (w_{(TiCl4)} = 400 g/l). Die Dosierung wird beim Erreichen des gewünschten koloristischen Endpunkts unterbrochen. Der pH-Wert wird hierbei mit Natronlauge konstant gehalten und anschließend auf 3,2 eingestellt. Im nächsten Schritt werden innerhalb von 60 Minuten 100 g einer FeCl₃-Lösung (w_{(Fe)}=0,10) zugesetzt, dann wird weiterhin soviel FeCl₃-Lösung hinzugefügt bis der gewünschte Farbton erreicht wird. Der pH-Wert wird hierbei mit NaOH konstant gehalten. Nach Ende der Dosierung der FeCl₃-Lösung wird der pH auf 8,0 hochgestellt. Es erfolgt innerhalb einer Stunde die Zugabe von 30 g Natronwasserglas-Lösung (w_{(SiO2)} = 0,2). Hierbei wird der pH-Wert durch Zugabe von Salzsäure konstant bei 8,0 gehalten. Anschließend wird der pH-Wert mit Salzsäure auf 5,0 eingestellt. Nach Ende der Dosierung der Natronwasserglas-Lösung wird die Pigmentsuspension über eine Saugnutsche filtriert. Mit VE-Wasser werden die Salzrückstände ausgewaschen. Es folgt die Trocknung über Nacht bei 110 °C. Anschließend wird das Pigment 30 Minuten bei 700 °C geglüht.

### Anwendungsbeispiele

### Beispiel A1: Duschgel

| **Rohstoff** | | **INCI** | **[%]** |
|---|---|---|---|
| **A** | | | |
| Ronastar® Golden Sparks | (1) | CALCIUM ALUMINUM BOROSILICATE, SILICA, CI 77891 (TITANIUM DIOXIDE), TIN OXIDE | 0,05 |
| Pigment nach Beispiel 1 | (1) | | 0,10 |
| Keltrol CG-SFT | (2) | XANTHAN GUM | 1,10 |
| Wasser, demineralisiert | | WATER, AQUA (WATER) | 54,90 |

| **B** | | | |
|---|---|---|---|
| Plantacare 2000 UP | (3) | DECYL GLUCOSIDE | 20,00 |
| Texapon ASV 50 | (3) | SODIUM LAURETH SULFATE, SODIUM LAURETH-8, SULFATE, MAGNESIUM LAURETH SULFATE, MAGNESIUM LAURETH-8 SULFATE, SODIUM OLETH, SULFATE, MAGNESIUM OLETH SULFATE | 3,60 |
| Bronidox L | (3) | PROPYLENE GLYCOL 5-BROMO-5-NITRO-1,3-DIOXANE | 0,30 |
| Frag 280851 Fruit Cocktail | (4) | PARFUM | 0,20 |
| 0,1 % Sicovit Chinolingelb 70 E 104 in Wasser | (5) | AQUA (WATER), WATER, CI 47005 (ACID YELLOW 3), ACID YELLOW 3 | 8,30 |
| 0,1 % Dragocolor Edelblau in Wasser | (6) | AQUA (WATER), WATER, CI 42090 (FD&C BLUE NO. 1), FD&C BLUE NO. 1 | 1,30 |

| **C** | | | |
|---|---|---|---|
| Citronensäure Monohydrat | (1) | CITRIC ACID | 0,15 |
| Wasser, demineralisiert | | WATER, AQUA (WATER) | 10,00 |

### Herstellung:

Phase A: Wasser im Kessel vorlegen und das Pigment einrühren. Keltrol CG-SFT unter Rühren langsam einstreuen und rühren bis es vollständig gelöst ist (nicht homogenisieren). Die Bestandteile der Phase B einzeln zur Phase A zufügen. Citronensäure Monohydrat in Wasser lösen und zum Ansatz geben und langsam rühren bis alles homogen verteilt ist. Den pH-Wert unter Zugabe von Citronensäure (nach Bedarf) auf 6,0 - 6,5 einstellen.

### Bezugsquellen:

(1) Merck KGaA/Rona®
(2) C. P. Kelco
(3) Cognis GmbH
(4) Drom
(5) BASF AG
(6) Symrise

### Beispiel A2: Lidschatten

| **Rohstoff** | | **INCI** | **[%]** |
|---|---|---|---|
| **A** | | | |
| Pigment nach Beispiel 4 | (1) | | 25,00 |
| Timiron® Splendid Gold | (1) | CI 77891(TITANIUM DIOXIDE), MICA, SILICA | 5,00 |
| Talkum | (1) | TALC | 49,50 |
| Kartoffelstärke | (2) | POTATO STARCH, SOLANUM TUBEROSUM (POTATO STARCH) | 7,50 |
| Magnesiumstearat | (1) | MAGNESIUM STEARATE | 2,50 |

| **B** | | | |
|---|---|---|---|
| Isopropylstearat | (3) | ISOPROPYL STEARATE | 9,34 |
| Cetylpalmitat | (1) | CETYL PALMITATE | 0,53 |
| Ewalin 1751 | (4) | PETROLATUM | 0,53 |
| Parfümöl Elegance + 79228 D MF | (5) | PARFUM | 0,20 |
| Propyl-4-hydroxybenzoat | (1) | PROPYLPARABEN | 0,10 |

### Herstellung:

Bestandteile der Phase A zusammen geben und vormischen. Anschließend die Pudermischung unter Rühren tropfenweise mit der geschmolzenen Phase B versetzen. Die Puder werden in Puderpfannen mit großem Durchmesser gefüllt und bei 80 bar verpreßt.

### Bezugsquellen:

(1) Merck KGaA/Rona®
(2) Suedstaerke GmbH
(3) Cognis GmbH
(4) H. Erhard Wagner GmbH
(5) Symrise

### Beispiel A3: Tagescreme (O/W)

| **Rohstoff** | | **INCI** | **[%]** |
|---|---|---|---|
| **A** | | | |
| Ronasphere® LDP | (1) | SILICA, CI 77891 (TITANIUM DIOXIDE), CI 77491 (IRON OXIDES) | 5,00 |
| Pigment nach Beispiel 1 | (1) | | 0,10 |
| Veegum HV | (2) | MAGNESIUM ALUMINUM SILICATE | 1,00 |
| Karion F flüssig | (1) | SORBITOL | 3,00 |
| Methyl-4-hydroxybenzoat | (1) | METHYLPARABEN | 0,18 |
| Wasser, demineralisiert | | AQUA (WATER) | 56,34 |

| **B** | | | |
|---|---|---|---|
| Arlacel 165 VP | (3) | GLYCERYL STEARATE, PEG-100 STEARATE | 5,00 |
| Lanette O | (4) | CETEARYL ALCOHOL | 1,50 |
| Miglyol 812 N | (5) | CAPRYLIC/CAPRIC TRIGLYCERIDE | 7,00 |
| Sheabutter fest | (6) | BUTYROSPERMUM PARKII (SHEA BUTTER) | 2,00 |
| Cetiol SN | (4) | CETEARYL ISONONANOATE | 7,00 |
| Eutanol G | (4) | OCTYLDODECANOL | 7,50 |
| Emulgade PL 68/50 | (4) | CETEARYL ALCOHOL, CETEARYL GLUCOSIDE | 2,00 |
| Propyl-4-hydroxybenzoat | (1) | PROPYLPARABEN | 0,08 |

| **C** | | | |
|---|---|---|---|
| Parfümöl 200 530 | (7) | PARFUM | 0,20 |
| Dow Corning 345 | (8) | CYCLOMETHICONE | 2,00 |
| Euxyl K 400 | (9) | PHENOXYETHANOL, METHYLDIBROMO, GLUTARONITRILE | 0,10 |
| Citronensäure Monohydrat | (1) | CITRIC ACID | 0,00 |

### Herstellung:

Phase B erwärmen bis die Lösung klar ist. Veegum im Wasser der Phase A dispergieren, restliche Rohstoffe zugeben, auf 80 °C erhitzen und Phase B zugeben. Phase A/B homogenisieren. Unter Rühren auf 40 °C abkühlen und Phase C zugeben. Auf Raumtemperatur abkühlen und pH 6,0 einstellen.

### Bezugsquellen:

(1) Merck KGaA/Rona®
(2) Vanderbilt
(3) Uniqema
(4) Cognis GmbH
(5) Sasol Germany GmbH
(6) H. Erhard Wagner GmbH
(7) Fragrance Resources
(8) Dow Corning
(9) Schülke & Mayr GmbH

### Beispiel A4: Sparkling Body Cream (OW)

| **Rohstoff** | | **INCI** | **[%]** |
|---|---|---|---|
| **A** | | | |
| Ronastar® Golden Sparks | (1) | CALCIUM ALUMINUM BOROSILICATE, SILICA, CI 77891 (TITANIUM DIOXIDE), TIN OXIDE | 1,00 |
| Pigment nach Beispiel 3 | (1) | | 1,00 |
| Wasser, demineralisiert | | WATER, AQUA (WATER) | 40,60 |
| Carbopol Ultrez 21 | (2) | ACRYLATES/C10-30 ALKYL ACRYLATE CROSSPOLYMER | 0,60 |
| Citronensäure Monohydrat | (1) | CITRIC ACID | 0,00 |

| **B** | | | |
|---|---|---|---|
| Wasser, demineralisiert | | WATER, AQUA (WATER) | 26,35 |
| 1,2-Propandiol | (1) | PROPYLENE GLYCOL | 3,00 |
| RonaCare® Allantoin | (1) | ALLANTOIN | 0,20 |

| **C** | | | |
|---|---|---|---|
| Paraffin flüssig | (1) | PARAFFINUM LIQUIDUM (MINERAL | 10,00 |

| **Rohstoff** | | **INCI** | **[%]** |
|---|---|---|---|
| | | OIL), MINERAL OIL | |
| Cetiol V | (3) | DECYL OLEATE | 6,00 |
| Hostaphat KL 340 D | (4) | TRILAURETH-4 PHOSPHATE | 3,00 |
| Cetylalkohol | (1) | CETYL ALCOHOL | 2,00 |
| Phenonip | (5) | PHENOXYETHANOL, BUTYLPARABEN, ETHYLPARABEN, PROPYLPARABEN, METHYLPARABEN | 0,50 |

| **D** | | | |
|---|---|---|---|
| Wasser, demineralisiert | | WATER, AQUA (WATER) | 3,50 |
| Triethanolamin | | TRIETHANOLAMINE | 0,35 |

| **E** | | | |
|---|---|---|---|
| Germall 115 | (6) | IMIDAZOLIDINYL UREA | 0,30 |
| Parfümöl Vogue | (7) | PARFUM | 0,10 |
| Wasser, demineralisiert | | WATER, AQUA (WATER) | 1,50 |

### Herstellung:

Das Perlglanzpigment im Wasser der Phase A dispergieren. Eventuell mit einigen Tropfen Citronensäure ansäuern, um die Viskosität zu vermindern. Carbopol unter Rühren einstreuen. Nach vollständiger Lösung die vorgelöste Phase B langsam einrühren. Phase A/B und Phase C auf 80 °C erhitzen, Phase C in Phase A/B einrühren, homogenisieren mit Phase D neutralisieren, nochmals homogenisieren und unter Rühren abkühlen. Bei 40 °C im Wasser der Phase E Germall 115 lösen, unter Rühren hinzugeben. Danach Parfümöl zugeben und unter Rühren auf Raumtemperatur abkühlen.

### Bezugsquellen:

(1) Merck KGaA/Rona®
(2) Noveon
(3) Cognis GmbH
(4) Clariant GmbH
(5) Nipa Laboratorien GmbH
(6) ISP Global Technologies
(7) Drom

### Beispiel A5: Creamy Eye Shadow

| **Rohstoff** | | **INCI** | **[%]** |
|---|---|---|---|
| **A** | | | |
| Pigment nach Beispiel 2 | (1) | | 20,00 |
| Micronasphere® M | (1) | MICA, SILICA | 6.00 |
| Unipure Green LC 789 CF | (2) | Cl 77289 (CHROMIUM HYDROXIDE GREEN) | 4,00 |

| **B** | | | |
|---|---|---|---|
| Crodamol PMP | (3) | PPG-2 MYRISTYL ETHER PROPIONATE | 37,80 |
| Syncrowax HGLC | (3) | C18-36 ACID TRIGLYCERIDE | 10,00 |
| Syncrowax HRC | (3) | TRIBEHENIN | 3,00 |
| Miglyol 812 N | (4) | CAPRYLIC/CAPRIC TRIGLYCERIDE | 14,00 |
| Stearinsäure | (1) | STEARIC ACID | 3,00 |
| Antaron V-216 | (5) | PVP/HEXADECENE COPOLYMER | 2,00 |
| Oxynex® K flüssig | (1) | PEG-8, TOCOPHEROL, ASCORBYL PALMITATE, ASCORBIC ACID, CITRIC ACID | 0,10 |
| Propyl-4-hydroxybenzoat | (1) | PROPYLPARABEN | 0,10 |

### Herstellung:

Phase B auf ca. 80 °C erhitzen bis alles geschmolzen ist und auf 65 °C abkühlen. Unter Rühren werden nun das Perlglanzpigment, Micronasphere und das vermahlene Chromoxid der Phase A zugegeben. Der Lidschatten wird bei 65 °C abgefüllt.

### Bezugsquellen,

(1) Merck KGaA/Rona®
(2) Les Colorants Wackherr
(3) Croda GmbH
(4) Sasol Germany GmbH
(5) ISP Global Technologies

### Beispiel A6: Hair Styling Gel

| **Rohstoff** | | **INCI** | **[%]** |
|---|---|---|---|
| **A** | | | |
| Ronastar® Golden Sparks | (1) | CALCIUM ALUMINUM BOROSILICATE, CI 77891 (TITANIUM DIOXIDE), SILICA, TIN OXIDE | 2,55 |
| Pigment nach Beispiel 1 | (1) | | 0,10 |
| Carbopol Ultrez 21 | (2) | ACRYLATES/C10-30 ALKYL ACRYLATE CROSSPOLYMER | 0,90 |
| Wasser, demineralisiert | | WATER, AQUA (WATER) | 50,35 |

| **B** | | | |
|---|---|---|---|
| Luviskol K 30 Pulver | (3) | PVP | 2,00 |
| Germaben II | (4) | PROPYLENE GLYCOL, DIAZOLIDINYL UREA, METHYLPARABEN, PROPYLPARABEN | 1,00 |
| Triethanolamin reinst | (1) | TRIETHANOLAMINE | 2,16 |
| Wasser, demineralisiert | | WATER, AQUA (WATER) | 40,94 |

### Herstellung:

Die Perlglanzpigmente im Wasser der Phase A dispergieren und das Carbopol unter Rühren einstreuen. Nach vollständiger Lösung die vorgelöste Phase B langsam einrühren.

### Bezugsquellen:

(1) Merck KGaA/Rona®
(2) Noveon
(3) BASF AG
(4) ISP Global Technologies

### Beispiel A7: Shampoo

| **Rohstoff** | | **INCI** | **[%]** |
|---|---|---|---|
| **A** | | | |
| Pigment nach Beispiel 4 | (1) | | 0,20 |
| Carbopol ETD 2020 | (2) | ACRYLATES/C10-30 ALKYL ACRYLATE CROSSPOLYMER | 0,90 |
| Wasser, demineralisiert | | AQUA (WATER) | 63,40 |

| **B** | | | |
|---|---|---|---|
| Triethanolamin reinst | (1) | TRIETHANOLAMINE | 0,90 |
| Wasser, demineralisiert | | AQUA (WATER) | 10,00 |

| **C** | | | |
|---|---|---|---|
| Plantacare 2000 UP | (3) | DECYL GLUCOSIDE | 20,00 |
| Texapon ASV 50 | (3) | SODIUM LAURETH SULFATE, SODIUM LAURETH-8 SULFATE, MAGNESIUM LAURETH SULFATE, MAGNESIUM LAURETH-8 SULFATE, SODIUM OLETH SULFATE, MAGNESIUM OLETH SULFATE | 4,35 |
| Bronidox L | (3) | PROPYLENE GLYCOL, 5-BROMO-5-NITRO-1,3-DIOXANE | 0,20 |
| Parfümöl 200 524 | (4) | PARFUM | 0,05 |
| Farbstofflösung (q.s.) | | | 0,00 |

### Herstellung:

Für Phase A das Pigment in das Wasser einrühren. Mit einigen Tropfen Citronensäure (10 %ig) ansäuern um die Viskosität zu vermindern und das Carbopol unter Rühren langsam einstreuen. Nach vollständiger Lösung langsam Phase B zugeben. Nacheinander werden nun die Bestandteile der Phase C zugegeben. Den pH-Wert auf 6,0 - 6,5 einstellen.

### Bezugsquellen:

(1) Merck KGaA/Rona®
(2) Noveon
(3) Cognis GmbH
(4) Fragrance Resources

### Beispiel A8: Shimmering Body Powder

| **Rohstoff** | | **INCI** | **[%]** |
|---|---|---|---|
| **A** | | | |
| Pigment nach Beispiel 1 | (1) | | 10,00 |

| **B** | | | |
|---|---|---|---|
| Microna® Matte Red | (1) | CI 77491 (IRON OXIDES), MICA | 1,00 |
| Microna® Matte Yellow | (1) | MICA, CI 77492 (IRON OXIDES) | 1,00 |
| Ronasphere® LDP | (1) | SILICA, CI 77891 (TITANIUM DIOXIDE), CI 77491 (IRON OXIDES) | 4,00 |
| Talkum | (1) | TALC | 25,00 |
| Glassflake Plättchen | (1) | CALCIUM ALUMINUM BOROSILICATE | 15,00 |
| Weißer Ton | (1) | KAOLIN | 14,70 |
| Mica M | (1) | MICA | 15,00 |
| Silk Mica | (1) | MICA | 9,50 |
| Propyl-4-hydroxybenzoat | (1) | PROPYLPARABEN | 0,30 |

| **C** | | | |
|---|---|---|---|
| Cetiol SQ | (2) | SQUALANE | 2,00 |
| Miglyol 812 N | (3) | CAPRYLIC/CAPRIC TRIGLYCERIDE | 2,00 |
| RonaCare® Tocopherolacetat | (1) | TOCOPHERYL ACETATE | 0,20 |
| Parfum | (4) | PARFUM | 0,30 |

### Herstellung:

Alle Bestandteile der Phase B zusammen einwiegen und einem Mixer homogen vermahlen. Anschließend Phase C zugeben und weiter mixen, dann Phase A zufügen und kurz vermahlen, bis das Effektpigment gleichmäßig verteilt ist.

### Bezugsquellen:

(1) Merck KGaA/Rona®
(2) Cognis GmbH
(3) Sasol Germany GmbH
(4) Symrise

### Beispiel A9: Long Lasting Lipgloss

| **Rohstoff** | | **INCI** | **[%]** |
|---|---|---|---|
| **A** | | | |
| Pigment nach Beispiel 1 | (1) | | 4,00 |
| Ronastar® Golden Sparks | (1) | CALCIUM ALUMINUM BOROSILICATE, SILICA, CI 77891 (TITANIUM DIOXIDE), TIN OXIDE | 6,00 |

| **B** | | | |
|---|---|---|---|
| Indopol H 100 | (2) | POLYBUTENE | 30,00 |
| Jojoba Glaze LV | (3) | SIMMONDSIA CHINENSIS (JOJOBA), JOJOBA, SEED OIL, ETHYLENE/PROPYLENE/STYRENE COPOLYMER, BUTYLENE/ETHYLENE/STYRENE COPOLYMER | 20,00 |
| Jojoba Glaze HV | (3) | SIMMONDSIA CHINENSIS (JOJOBA), JOJOBA, SEED OIL, ETHYLENE/PROPYLENE/STYRENE COPOLYMER, BUTYLENE/ETHYLENE/STYRENE COPOLYMER | 10,00 |
| Rizinusöl | (4) | CASTOR OIL, RICINUS COMMUNIS (CASTOR OIL) | 23,15 |
| Bienenwachs gebleicht | (1) | BEESWAX, CERA ALBA (BEESWAX) | 4,00 |
| Propyl-4-hydroxybenzoat | (1) | PROPYLPARABEN | 0,10 |
| Oxynex® K flüssig | (1) | PEG-8, TOCOPHEROL, ASCORBYL PALMITATE, ASCORBIC ACID, CITRIC ACID | 0,05 |
| Jaune Covapate W 1761 | (5) | RICINUS COMMUNIS (CASTOR OIL), CI 19140 (FD&C YELLOW No. 5 ALUMINUM LAKE) | 1,00 |

| **C** | | | |
|---|---|---|---|
| Neosil CT11 | (6) | SILICA | 1,50 |
| Fragrance Tendresse 75418C | (7) | PARFUM | 0,20 |

### Herstellung:

Alle Bestandteile der Phase B werden zusammen eingewogen, auf 80°C erhitzt und gut durchgerührt. Die Pigmente der Phase A unterrühren, das Neosil unter Rühren einstreuen und zum Schluß das Parfüm zugeben. Die homogene Mischung in Container abfüllen.

### Bezugsquellen:

(1) Merck KGaA/Rona®
(2) BP Lavera Sud
(3) Desert Whale
(4) Henry Lamotte GmbH
(5) Les Colorants Wackherr
(6) Ineos Silicas Limited
(7) Symrise

### Beispiel A10: Nagellack

| **Rohstoff** | | **INCI** | **[%]** |
|---|---|---|---|
| Pigment nach Beispiel 4 | (1) | | 1,75 |
| Ronastar® Golden Sparks | (1) | CALCIUM ALUMINUM BOROSILICATE, SILICA, CI 77891 (TITANIUM DIOXIDE), TIN OXIDE | 0,25 |
| Thixotrope Nagellack-Base 155 | (2) | BUTYL ACETATE, ETHYL ACETATE, NITROCELLULOSE, ACETYL TRIBUTYL CITRATE, PHTHALIC ANHYDRIDE/TRIMELLITIC ANHYDRIDE/GLYCOLS COPOLYMER, ISOPROPYL ALCOHOL, STEARALKONIUM HECTORITE, ADIPIC ACID/FUMARIC ACID/PHTHALIC ACID/TRICYCLODECANE DIMETHANOL COPOLYMER, CITRIC ACID | 98,00 |

### Herstellung:

Die Pigmente werden zusammen mit der Lackbase eingewogen, gut mit einem Spatel von Hand vermischt und anschließend 10 min bei 1000 Upm gerührt.

### Bezugsquellen:

(1) Merck KGaA/Rona®
(2) Durlin/Bergerac NC

### Beispiel A11: Volume Mascara (O/W)

| **Rohstoff** | | **INCI** | **[%]** |
|---|---|---|---|
| **A** | | | |
| Mica Black | (1) | CI 77499 (IRON OXIDES), MICA, CI 77891 (TITANIUM DIOXIDE) | 5,00 |
| Colorona® Red Brown | (1) | MICA, CI 77491 (IRON OXIDES), CI 77891 (TITANIUM DIOXIDE) | 3,00 |
| Pigment nach Beispiel 2 | (1) | | 2,00 |
| Satin Mica | (1) | MICA | 2,00 |

| **B** | | | |
|---|---|---|---|
| Dermacryl 79 | (2) | ACRYLATES/OCTYLACRYLAMIDE COPOLYMER | 3,50 |
| Bienenwachs gebleicht | (1) | BEESWAX, CERA ALBA (BEESWAX) | 3,00 |
| Syncrowax HRC | (3) | TRIBEHENIN | 3,50 |
| Stearinsäure | (1) | STEARIC ACID | 5,00 |
| Tegin M | (4) | GLYCERYL STEARATE | 3,50 |
| Tegosoft CT | (4) | CAPRYLIC/CAPRIC TRIGLYCERIDE | 2,50 |
| Dow Corning 556 | (5) | PHENYL TRIMETHICONE | 2,00 |
| RonaCare® Tocopherolacetat | (1) | TOCOPHERYL ACETATE | 0,50 |
| Phenonip | (6) | PHENOXYETHANOL, BUTYLPARABEN, ETHYLPARABEN, PROPYLPARABEN, METHYLPARABEN | 0,80 |

| **C** | | | |
|---|---|---|---|
| Wasser, demineralisiert | | WATER, AQUA (WATER) | 59,15 |
| AMP Ultra PC 1000 | (7) | AMINOMETHYL PROPANOL | 1,25 |
| 1,3-Butandiol | (1) | BUTYLENE GLYCOL | 1,00 |
| RonaCare® Biotin Plus | (1) | UREA, DISODIUM PHOSPHATE, BIOTIN, CITRIC ACID | 0,50 |

| **D** | | | |
|---|---|---|---|
| Germall 115 | (8) | IMIDAZOLIDINYL UREA | 0,30 |
| Wasser, demineralisiert | | WATER, AQUA (WATER) | 1,50 |

### Herstellung:

Alle Bestandteile der Phase B außer Demacryl 79 zusammen bei ca. 85 °C aufschmelzen, unter rühren Demacryl 79 zugeben und 20 min rühren lassen bis alles homogen verteilt ist. Die Bestandteile der Phase C auf ca 85 °C erhitzen. Die Perlglanzpigmente der Phase A in Phase C einrühren. Phase C zu Phase B geben, weiter rühren und 1 min mit dem Ultra-Turrax T25 bei 8000 upm homogenisieren. Unter rühren abkühlen lassen und bei 40 °C Phase D zufügen.

### Bezugsquellen:

(1) Merck KGaA/Rona®
(2) National Starch & Chemical
(3) Croda GmbH
(4) Degussa-Goldschmidt AG
(5) Dow Corning
(6) Nipa Laboratorien GmbH
(7) Angus Chemie GmbH
(8) ISP Global Technologies

### Beispiel A12: Getönte Tagescreme mit UV-Schutz (O/W)

| **Rohstoff** | | **INCI** | **[%]** |
|---|---|---|---|
| **A** | | | |
| Eusolex® 2292 | (1) | ETHYLHEXYL METHOXYCINNAMATE, BHT | 3,00 |
| Eusolex® 4360 | (1) | BENZOPHENONE-3 | 3,00 |
| Arlacel 165 VP | (2) | GLYCERYL STEARATE, PEG-100 STEARATE | 5,00 |
| Eusolex® HMS | (1) | HOMOSALATE | 5,00 |
| Arlacel 165 VP | (2) | GLYCERYL STEARATE, PEG-100 STEARATE | 3,00 |
| Montanov 68 | (3) | CETEARYL ALCOHOL, CETEARYL GLUCOSIDE | 3,00 |
| Dow Corning 345 | (4) | CYCLOMETHICONE | 0,50 |
| Eutanol G | (5) | OCTYLDODECANOL | 2,00 |
| Propyl-4-hydroxybenzoat | (1) | PROPYLPARABEN | 0,05 |

| **B** | | | |
|---|---|---|---|
| Eusolex® T-2000 | (1) | TITANIUM DIOXIDE, ALUMINA, SIMETHICONE | 3,00 |
| Extender W | (1) | MICA, CI 77891 (TITANIUM DIOXIDE) | 4,00 |
| Microna® Matte Yellow | (1) | MICA, CI 77492 (IRON OXIDES) | 2,00 |
| Microna® Matte Orange | (1) | MICA, CI 77491 (IRON OXIDES) | 0,20 |
| Microna® Matte Red | (1) | CI 77491 (IRON OXIDES), MICA | 0,20 |
| Microna® Matte Black | (1) | CI 77499 (IRON OXIDES), MICA | 0,20 |
| Pigment nach Beispiel 3 | (1) | | 2,00 |
| Karion FP, flüssig | (1) | SORBITOL | 5,00 |
| RonaCare® Allantoin | (1) | ALLANTOIN | 0,50 |
| Keltrol T | (6) | XANTHAN GUM | 0,20 |
| Chemag 2000 | (7) | IMIDAZOLIDINYL UREA | 0,30 |
| Euxyl K 400 | (8) | PHENOXYETHANOL, METHYLDIBROMO GLUTARONITRILE | 0,10 |
| Methyl-4-hydroxybenzoat | (1) | METHYLPARABEN | 0,15 |
| Wasser, demineralisiert | | AQUA (WATER) | 57,60 |

### Herstellung:

Alle Bestandteile außer Keltrol T im Wasser von Phase B dispergieren. Das Keltrol unter Rühren in Phase B einstreuen und nach 15 Minuten auf 80°C erhitzen. Phase A auf 75 °C erhitzen. Langsam Phase B in Phase A einrühren und homogenisieren. Unter Rühren abkühlen.

### Bezugsquellen:

(1) Merck KGaA/Rona®
(2) Uniqema
(3) Seppic
(4) Dow Corning
(5) Cognis GmbH
(6) C. P. Kelco
(7) Chemag AG
(8) Schülke & Mayr GmbH

### Beispiel A13: Cream conditioner

| **Rohstoff** | | **INCI** | **%** |
|---|---|---|---|
| **A** | | | |
| Water | | Aqua (water) | 79.7 |
| Pigment nach Beispiel 3 | (1) | | 0.50 |
| Luviquat Hold | (2) | Polyquaternium-46 | 5.00 |
| Luviquat PQ 11 | (2) | Polyquaternium-11 | 2.00 |
| 1,3-Butandiol | (1) | Butylene Glycol | 3.00 |

| **B** | | | |
|---|---|---|---|
| Cremophor A 6 | (2) | Ceteareth-6 and Stearyl Alcohol | 3.00 |
| Ammonyx 4 | (2) | Stearalkonium Chloride | 3.00 |
| Lanette Wax O | (3) | Cetearyl Alcohol | 2.00 |
| Eusolex 2292 | (1) | Octyl Methoxycinnamate | 0.10 |

| **C** | | | |
|---|---|---|---|
| RonaCare® Tocopherol acetate | (1) | Tocopheryl acetate | 0.50 |
| RonaCare® Bisabolol nat. | (1) | Bisabolol | 0.10 |
| Parfum | | Parfum | 0.10 |
| Germaben II | (4) | Propylene glycol, Diazolidinyl urea, Methylparaben, Propylparaben | 1.00 |

### Herstellung:

Die Pigmente in Wasser der Phase A dispergieren und die restlichen Rohstoffe zugeben. Nach jeder Zugabe durchrühren und anschließend auf 75 °C erhitzen. Die Rohstoffe der Phase B mischen, auf 75 - 80 °C erhitzen und zu Phase A dazugeben. Mischen bis eine homogene Verteilung vorliegt. Phase C bei 45 °C zugeben.

### Bezugsquellen:

(1) Merck KGaA/Rona®
(2) BASF AG
(3) Cognis GmbH
(4) ISP Global Technologies

### Beispiel A14: Herstellung von Hartkaramellen

| **Rohstoff** | | | **%** |
|---|---|---|---|
| Zucker | (3) | | 41,0 |
| Water | | Aqua (Water) | 17,118 |
| Gluckosesirup | (2) | C* Sweet | 41,0 |
| Pigment nach Beispiel 1 | (1) | (0,1 % bezogen auf die Gießmasse) | 0,082 |
| E 104 1 : 100 verd. | (4) | Sikovit | 0,4 |
| Aroma | (5) | Banane 9/030388 | 0,4 |

### Bezugsquellen:

(1) Fa. Merck KGaA
(2) Fa. Cerestar, Krefeld
(3) Fa. Südzucker
(4) Fa. BASF, Ludwigshafen
(5) Fa. Dragaco, Holzminden

Der Zucker wird mit dem Wasser auf 100 °C erhitzt und danach mit Glucosesirup versetzt. Die Lösung wird anschließend auf 145 °C erhitzt. Nach Zugabe des Goldpigments, der Farblösung und dem Aroma wird die Karamell-Lösung mit einem Gießtrichter in gefettete Formen gegossen. Zuletzt lässt man zwei Stunden abkühlen. Das Goldpigment kann sowohl mit dem Zucker vermischt werden als auch mit dem Glukosesirup vermischt zugegeben werden. Diese Variante enthält keine Säure, da hierdurch die Karamelisation zu stark würde.

### Beispiel A15: Coating von Tabletten

### a) Einwaage 1 kg weiße Tabletten d=8mm, G=200mg

| **Rohstoff** | | | % |
|---|---|---|---|
| Sepifilm Lp 10 | (3) | Gemisch aus Hydroxypropylmedthylcellulose, Stearinsäure und mikrokristalliner Cellulose | 6,0 |
| Pigment nach Beispiel 1 | (1) | | 5,0 |
| Water | | Aqua (Water) | 89,0 |

### Bezugsquellen:

(1) Fa. Merck KGaA
(2) Fa. Seppic

Gesamtauftragsmenge: 200 g
Dies entspricht 1,2 mg Polymer/cm² Tablettenoberfläche

Herstellung der Film-Coating-Lösung:
- Das Goldpigment wird in Wasser eingerührt. Anschließend fügt man zusätzliche Farbstoffe hinzu. Schließlich streut man den Filmbildner (HPMC) in die Suspension ein. Durch die ansteigende Viskosität bedingt, muss auch die Rührgeschwindigkeit dementsprechend erhöht werden. Nach ca. 40-60 Minuten ist die HPMC vollständig gelöst und die Lösung kann nun auf die Tabletten aufgesprüht werden.
- Der Sprühauftrag erfolgt mittels gängigem Standard-Coating-Verfahren.

## Patentansprüche

1. Pigmente auf der Basis von mehrfach beschichteten plättchenförmigen Substraten, **dadurch gekennzeichnet, dass** auf dem Substrat
(A0) optional eine Schicht aus SiO₂,
(A) eine farblose Beschichtung mit einem Brechungsindex n < 1,8, wobei die Schicht (A) chemisch nicht identisch ist mit der Schicht (A0),
(B) eine farblose Beschichtung mit einem Brechungsindex n ≥ 1,8, wobei die Schicht (B) Dicken von 1-15 nm aufweist,
(C) eine farblose Beschichtung mit einem Brechungsindex n ≥ 1,8, wobei die Schicht (C) chemisch nicht identisch ist mit der Schicht (B),
(D) eine farbige Beschichtung mit einem Brechungsindex n ≥ 1,8 und optional
(E) eine äußere Schutzschicht,
ist.

2. Pigmente nach Anspruch 1, **dadurch gekennzeichnet, dass** es sich bei den plättchenförmigen Substraten um natürlichen oder synthetischen Glimmer, Talkum, Kaolin, plättchenförmige Eisen- oder Aluminiumoxide, Glas-, SiO₂-, TiO₂-, Graphitplättchen, synthetische trägerfreie Plättchen, Titannitrid, Titansilicid, Liquid crystal polymers (LCPs), holographische Pigmente, BiOCl und plättchenförmige Mischoxide oder deren Gemische handelt.

3. Pigmente nach Anspruch 1 oder 2, **dadurch gekennzeichnet, dass** es sich bei den plättchenförmigen Substraten um Glasplättchen, natürliche Glimmerplättchen, synthetische Glimmerplättchen oder Aluminiumoxidplättchen handelt.

4. Pigmente nach einem oder mehreren der Ansprüche 1 bis 3, **dadurch gekennzeichnet, dass** es sich bei den plättchenförmigen Substraten, um Glasplättchen oder synthetische Glimmerplättchen handelt.

5. Pigmente nach einem oder mehreren der Ansprüche 1 bis 4, **dadurch gekennzeichnet, dass** die Glasplättchen aus Fensterglas, Calciumalumiumborosilikat-Glas, A-Glas, C-Glas, E-Glas, ECR-Glas, Duran®-Glas, Laborgeräteglas, bestehen.

6. Pigmente nach einem oder mehreren der Ansprüche 1 bis 5, **dadurch gekennzeichnet, dass** die Glasplättchen aus Calciumalumiumborosilikat-Glas, C-Glas, E-Glas oder ECR-Glas bestehen.

7. Pigmente nach einem oder mehreren der Ansprüche 1 bis 6, **dadurch gekennzeichnet, dass** die Schicht (A0) mit Rußpartikeln, Metallpartikeln und/oder Farbpigmenten dotiert ist.

8. Pigmente nach einem oder mehreren der Ansprüche 1 bis 7, **dadurch gekennzeichnet, dass** die Schichten (A) bis (D) aus Oxiden bestehen.

9. Pigmente nach einem oder mehreren der Ansprüche 1 bis 8 **dadurch gekennzeichnet, dass** die Oxide ausgewählt sind aus der Gruppe Al₂O₃, TiO₂, ZrO₂, SnO₂, ZnO, Ce₂O₃, Fe₂O₃, Fe₃O₄, FeOOH, Cr₂O₃, CoO, Co₃O₄, SiO₂, VO₂, V₂O₃, NiO, Titansuboxiden oder deren Gemische.

10. Pigmente nach einem oder mehreren der Ansprüche 1 bis 9 **dadurch gekennzeichnet, dass** sie zu Erhöhung der Licht-, Temperatur- und Wetterstabilität eine äußere Schutzschicht (E) aufweisen.

11. Pigmente nach einem oder mehreren der Ansprüche 1 bis 10, **dadurch gekennzeichnet, daß** zwischen der Schicht (C) und (D) eine weitere Zwischenschicht (C0) vorhanden ist, die aus den Komponenten der Schichten (C) und (D) besteht.

12. Pigmente nach einem oder mehreren der Ansprüche 1 bis 11 **dadurch gekennzeichnet, dass** die Pigmente folgenden Schichtaufbau besitzen:
Glimmerplättchen (natürlich) + SiO₂ + SnO₂ + TiO₂ + Fe₂O₃
Glimmerplättchen (natürlich) + Al₂O₃ + SnO₂ + TiO₂ + Fe₂O₃
Glimmerplättchen (natürlich) + SiO₂ + Al₂O₃ + SnO₂ + TiO₂ + Fe₂O₃
Glimmerplättchen (synthetisch) + SiO₂ + SnO₂ + TiO₂ + Fe₂O₃
Glimmerplättchen (synthetisch) + Al₂O₃ + SnO₂ + TiO₂ + Fe₂O₃
Glimmerplättchen (synthetisch) + SiO₂ + Al₂O₃ + SnO₂ + TiO₂ + Fe₂O₃
Glimmerplättchen (natürlich) + SiO₂ + SnO₂ + TiO₂ + TiO₂/Fe₂O₃ + Fe₂O₃
Glimmerplättchen (natürlich) + Al₂O₃ + SnO₂ + TiO₂ + TiO₂/Fe₂O₃ + Fe₂O₃
Glimmerplättchen (natürlich) + SiO₂ + Al₂O₃ + SnO₂ + TiO₂ + TiO₂/Fe₂O₃ + Fe₂O₃
Glimmerplättchen (synthetisch) + SiO₂ + SnO₂ + TiO₂ + TiO₂/Fe₂O₃ + Fe₂O₃
Glimmerplättchen (synthetisch) + Al₂O₃ + SnO₂ + TiO₂ + TiO₂/Fe₂O₃ + Fe₂O₃
Glimmerplättchen (synthetisch) + SiO₂ + Al₂O₃ + SnO₂ + TiO₂ + TiO₂/Fe₂O₃ + Fe₂O₃
Glasplättchen + SiO₂ + SnO₂ + TiO₂ + Fe₂O₃
Glasplättchen + Al₂O₃ + SnO₂ + TiO₂ + Fe₂O₃
Glasplättchen + SiO₂ + Al₂O₃ + SnO₂ + TiO₂ + Fe₂O₃
Glasplättchen + SiO₂ + SnO₂ + TiO₂ + TiO₂/Fe₂O₃ + Fe₂O₃
Glasplättchen + Al₂O₃ + SnO₂ + TiO₂ + TiO₂/Fe₂O₃ + Fe₂O₃
Glasplättchen + SiO₂ + Al₂O₃ + SnO₂ + TiO₂ + TiO₂/Fe₂O₃ + Fe₂O₃
Al₂O₃-Plättchen + SiO₂ + SnO₂ + TiO₂ + Fe₂O₃
Al₂O₃-Plättchen + Al₂O₃ + SnO₂ + TiO₂ + Fe₂O₃
Al₂O₃-Plättchen + SiO₂ + Al₂O₃ + SnO₂ + TiO₂ + Fe₂O₃
Al₂O₃-Plättchen + SiO₂ + SnO₂ + TiO₂ + TiO₂/Fe₂O₃ + Fe₂O₃
Al₂O₃-Plättchen + Al₂O₃ + SnO₂ + TiO₂ + TiO₂/Fe₂O₃ + Fe₂O₃
Al₂O₃-Plättchen + SiO₂ + Al₂O₃ + SnO₂ + TiO₂ + TiO₂/Fe₂O₃ + Fe₂O₃
Glimmerplättchen (natürlich) + SiO₂ + SnO₂ + TiO₂ + Fe₂O₃ + SiO₂
Glimmerplättchen (natürlich) + Al₂O₃ + SnO₂ + TiO₂ + Fe₂O₃ + SiO₂
Glimmerplättchen (natürlich) + SiO₂ + Al₂O₃ + SnO₂ + TiO₂ + Fe₂O₃ + SiO₂
Glimmerplättchen (synthetisch) + SiO₂ + SnO₂ + TiO₂ + Fe₂O₃ + SiO₂
Glimmerplättchen (synthetisch) + Al₂O₃ + SnO₂ + TiO₂ + Fe₂O₃ + SiO₂
Glimmerplättchen (synthetisch) + SiO₂ + Al₂O₃ + SnO₂ + TiO₂ + Fe₂O₃ + SiO₂
Glimmerplättchen (natürlich) + SiO₂ + SnO₂ + TiO₂ + TiO₂/Fe₂O₃ + Fe₂O₃ + SiO₂
Glimmerplättchen (natürlich) + Al₂O₃ + SnO₂ + TiO₂ + TiO₂/Fe₂O₃ + Fe₂O₃+ SiO₂
Glimmerplättchen (natürlich) + SiO₂ + Al₂O₃ + SnO₂ + TiO₂ + TiO₂/Fe₂O₃ + Fe₂O₃ + SiO₂
Glimmerplättchen (synthetisch) + SiO₂ + SnO₂ + TiO₂ + TiO₂/Fe₂O₃ + Fe₂O₃ + SiO₂
Glimmerplättchen (synthetisch) + Al₂O₃ + SnO₂ + TiO₂ + TiO₂/Fe₂O₃ + Fe₂O₃+ SiO₂
Glimmerplättchen (synthetisch) + SiO₂ + Al₂O₃ + SnO₂ + TiO₂ + TiO₂/Fe₂O₃ + Fe₂O₃+ SiO₂
Glasplättchen + SiO₂ + SnO₂ + TiO₂ + Fe₂O₃ + SiO₂
Glasplättchen + Al₂O₃ + SnO₂ + TiO₂ + Fe₂O₃ + SiO₂
Glasplättchen + SiO₂ + Al₂O₃ + SnO₂ + TiO₂ + Fe₂O₃+ SiO₂
Glasplättchen + SiO₂ + SnO₂ + TiO₂ + TiO₂/Fe₂O₃ + Fe₂O₃+ SiO₂
Glasplättchen + Al₂O₃ + SnO₂ + TiO₂ + TiO₂/Fe₂O₃ + Fe₂O₃ + SiO₂
Glasplättchen + SiO₂ + Al₂O₃ + SnO₂ + TiO₂ + TiO₂/Fe₂O₃ + Fe₂O₃ + SiO₂
Al₂O₃-Plättchen + SiO₂ + SnO₂ + TiO₂ + Fe₂O₃ + SiO₂
Al₂O₃-Plättchen + Al₂O₃ + SnO₂ + TiO₂ + Fe₂O₃ + SiO₂
Al₂O₃-Plättchen + SiO₂ + Al₂O₃ + SnO₂ + TiO₂ + Fe₂O₃ + SiO₂
Al₂O₃-Plättchen + SiO₂ + SnO₂ + TiO₂ + TiO₂/Fe₂O₃ + Fe₂O₃ + SiO₂
Al₂O₃-Plättchen + Al₂O₃ + SnO₂ + TiO₂ + TiO₂/Fe₂O₃ + Fe₂O₃ + SiO₂
Al₂O₃-Plättchen + SiO₂ + Al₂O₃ + SnO₂ + TiO₂ + TiO₂/Fe₂O₃ + Fe₂O₃ + SiO₂.

13. Verfahren zur Herstellung der Pigmente nach einem oder mehreren der Ansprüche 1 bis 12, **dadurch gekennzeichnet, dass** die Beschichtung der Substrate nasschemisch durch hydrolytische Zersetzung von Metallsalzen in wässrigem Medium oder im Wirbelbettreaktor durch Gasphasenbeschichtung erfolgt.

14. Verwendung der Pigmente nach einem oder mehreren der Ansprüche 1 bis 12 in Farben, Lacken, Automobillacken, Pulverlacken, Druckfarben, Sicherheitsdruckfarben, Kunststoffen, keramischen Materialien, Gläsern, Papier, in Tonern für elektrophotographische Druckverfahren, im Saatgut, in Gewächshausfolien und Zeltplanen, als Absorber bei der Lasermarkierung von Papier und Kunststoffen, in kosmetischen Formulierungen, zur Herstellung von Pigmentanteigungen mit Wasser, organischen und/oder wässrigen Lösemitteln, zur Herstellung von Pigmentpräparationen und Trockenpräparaten, zur Masseeinfärbung von Lebensmitteln, zur Einfärbung von Überzügen von Lebensmittelprodukten und Pharmaerzeugnissen und in Wertdokumenten.

15. Verwendung der Pigmente nach Anspruch 14 in Lippenstiften, Lipgloss, Rouge, Eyeliner, Lidschatten, (Volumen)Mascara, Nagellacken, Pflegecremes, Tagescremes, Nachtcremes, Körperlotionen, Reinigungsmilch, Körperpuder, Haargele, Haarlack, Haarmasken, Haarspülungen, Haarshampoos, Duschgelen, Duschölen, Badeölen, Sonnenschutz, Prä-Sun- und After-Sun-Präparate, Bräunungslotionen, Bräunungssprays, Make-ups, Lotions, Seifen, Badesalze, Zahnpasta, Gesichtsmasken, Presspuder, lose Puder und Gele.

16. Formulierungen enthaltend das Pigment nach einem oder mehreren der Ansprüche 1 bis 12.

17. Formulierungen nach Anspruch 16 enthaltend das Pigment nach einem oder mehreren der Ansprüche 1 bis 12 und mindestens einen Bestandteil ausgewählt aus der Gruppe der Absorptionsmittel, Adstringenzien, antimikrobiellen Stoffen, Antioxidantien, Antiperspirantien, Antischaummitteln, Antischuppenwirkstoffen, Antistatika, Bindemitteln, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen.

## Claims

1. Pigments based on multicoated flake-form substrates, **characterised in that**
(A0) optionally an SiO₂ layer,
(A) a colourless coating having a refractive index n < 1.8, where layer (A) is not chemically identical with layer (A0),
(B) a colourless coating having a refractive index n ≥ 1.8, where layer (B) has thicknesses of 1-15 nm,
(C) a colourless coating having a refractive index n ≥ 1.8, where layer (C) is not chemically identical with layer (B),
(D) a colourless coating having a refractive index n ≥ 1.8 and optionally
(E) an outer protective layer,
are on the substrate.

2. Pigments according to Claim 1, **characterised in that** the flake-form substrates are natural or synthetic mica, talc, kaolin, flake-form iron oxides or aluminium oxides, glass flakes, SiO₂ flakes, TiO₂ flakes, graphite flakes, synthetic support-free flakes, titanium nitride, titanium silicide, liquid crystal polymers (LCPs), holographic pigments, BiOCl and flake-form mixed oxides or mixtures thereof.

3. Pigments according to Claim 1 or 2, **characterised in that** the flake-form substrates are glass flakes, natural mica flakes, synthetic mica flakes or aluminium oxide flakes.

4. Pigments according to one or more of Claims 1 to 3, **characterised in that** the flake-form substrates are glass flakes or synthetic mica flakes.

5. Pigments according to one or more of Claims 1 to 4, **characterised in that** the glass flakes consist of window glass, calcium aluminium borosilicate glass, A glass, C glass, E glass, ECR glass, Duran® glass, laboratory equipment glass.

6. Pigments according to one or more of Claims 1 to 5, **characterised in that** the glass flakes consist of calcium aluminium borosilicate glass, C glass, E glass or ECR glass.

7. Pigments according to one or more of Claims 1 to 6, **characterised in that** layer (A0) is doped with carbon black particles, metal particles and/or coloured pigments.

8. Pigments according to one or more of Claims 1 to 7, **characterised in that** layers (A) to (D) consist of oxides.

9. Pigments according to one or more of Claims 1 to 8, **characterised in that** the oxides are selected from the group Al₂O₃, TiO₂, ZrO₂, SnO₂, ZnO, Ce₂O₃, Fe₂O₃, Fe₃O₄, FeOOH, Cr₂O₃, CoO, Co₃O₄, SiO₂, VO₂, V₂O₃, NiO, titanium suboxides or mixtures thereof.

10. Pigments according to one or more of Claims 1 to 9, **characterised in that** they have an outer protective layer (E) for increasing the light, temperature and weather stability.

11. Pigments according to one or more of Claims 1 to 10, **characterised in that** a further interlayer (C0) which consists of the components of layers (C) and (D) is present between layers (C) and (D).

12. Pigments according to one or more of Claims 1 to 11, **characterised in that** the pigments have the following layer structure:
mica flake (natural) + SiO₂ + SnO₂ + TiO₂ + Fe₂O₃
mica flake (natural) + Al₂O₃ + SnO₂ + TiO₂ + Fe₂O₃
mica flake (natural) + SiO₂ + Al₂O₃ + SnO₂ + TiO₂ + Fe₂O₃
mica flake (synthetic) + SiO₂ + SnO₂ + TiO₂ + Fe₂O₃
mica flake (synthetic) + Al₂O₃ + SnO₂ + TiO₂ + Fe₂O₃
mica flake (synthetic) + SiO₂ + Al₂O₃ + SnO₂ + TiO₂ + Fe₂O₃
mica flake (natural) + SiO₂ + SnO₂ + TiO₂ + TiO₂/Fe₂O₃ + Fe₂O₃
mica flake (natural) + Al₂O₃ + SnO₂ + TiO₂ + TiO₂/Fe₂O₃ + Fe₂O₃
mica flake (natural) + SiO₂ + Al₂O₃ + SnO₂ + TiO₂ + TiO₂/Fe₂O₃ + Fe₂O₃
mica flake (synthetic) + SiO₂ + SnO₂ + TiO₂ + TiO₂/Fe₂O₃ + Fe₂O₃
mica flake (synthetic) + Al₂O₃ + SnO₂ + TiO₂ + TiO₂/Fe₂O₃ + Fe₂O₃
mica flake (synthetic) + SiO₂ + Al₂O₃ + SnO₂ + TiO₂ + TiO₂/Fe₂O₃ + Fe₂O₃
glass flake + SiO₂ + SnO₂ + TiO₂ + Fe₂O₃
glass flake + Al₂O₃ + SnO₂ + TiO₂ + Fe₂O₃
glass flake + SiO₂ + Al₂O₃ + SnO₂ + TiO₂ + Fe₂O₃
glass flake + SiO₂ + SnO₂ + TiO₂ + TiO₂/Fe₂O₃ + Fe₂O₃
glass flake + Al₂O₃ + SnO₂ + TiO₂ + TiO₂/Fe₂O₃ + Fe₂O₃
glass flake + SiO₂ + Al₂O₃ + SnO₂ + TiO₂ + TiO₂/Fe₂O₃ + Fe₂O₃
Al₂O₃ flake + SiO₂ + SnO₂ + TiO₂ + Fe₂O₃
Al₂O₃ flake + Al₂O₃ + SnO₂ + TiO₂ + Fe₂O₃
Al₂O₃ flake + SiO₂ + Al₂O₃ + SnO₂ + TiO₂ + Fe₂O₃
Al₂O₃ flake + SiO₂ + SnO₂ + TiO₂ + TiO₂/Fe₂O₃ + Fe₂O₃
Al₂O₃ flake + Al₂O₃ + SnO₂ + TiO₂ + TiO₂/Fe₂O₃ + Fe₂O₃
Al₂O₃ flake + SiO₂ + Al₂O₃ + SnO₂ + TiO₂ + TiO₂/Fe₂O₃ + Fe₂O₃
mica flake (natural) + SiO₂ + SnO₂ + TiO₂ + Fe₂O₃ + SiO₂
mica flake (natural) + Al₂O₃ + SnO₂ + TiO₂ + Fe₂O₃ + SiO₂
mica flake (natural) + SiO₂ + Al₂O₃ + SnO₂ + TiO₂ + Fe₂O₃ + SiO₂
mica flake (synthetic) + SiO₂ + SnO₂ + TiO₂ + Fe₂O₃ + SiO₂
mica flake (synthetic) + Al₂O₃ + SnO₂ + TiO₂ + Fe₂O₃ + SiO₂
mica flake (synthetic) + SiO₂ + Al₂O₃ + SnO₂ + TiO₂ + Fe₂O₃ + SiO₂
mica flake (natural) + SiO₂ + SnO₂ + TiO₂ + TiO₂/Fe₂O₃ + Fe₂O₃ + SiO₂
mica flake (natural) + Al₂O₃ + SnO₂ + TiO₂ + TiO₂/Fe₂O₃ + Fe₂O₃ + SiO₂
mica flake (natural) + SiO₂ + Al₂O₃ + SnO₂ + TiO₂ + TiO₂/Fe₂O₃ + Fe₂O₃ + SiO₂
mica flake (synthetic) + SiO₂ + SnO₂ + TiO₂ + TiO₂/Fe₂O₃ + Fe₂O₃ + SiO₂
mica flake (synthetic) + Al₂O₃ + SnO₂ + TiO₂ + TiO₂/Fe₂O₃ + Fe₂O₃ + SiO₂
mica flake (synthetic) + SiO₂ + Al₂O₃ + SnO₂ + TiO₂ + TiO₂/Fe₂O₃ + Fe₂O₃ + SiO₂
glass flake + SiO₂ + SnO₂ + TiO₂ + Fe₂O₃ + SiO₂
glass flake + Al₂O₃ + SnO₂ + TiO₂ + Fe₂O₃ + SiO₂
glass flake + SiO₂ + Al₂O₃ + SnO₂ + TiO₂ + Fe₂O₃ + SiO₂
glass flake + SiO₂ + SnO₂ + TiO₂ + TiO₂/Fe₂O₃ + Fe₂O₃ + SiO₂
glass flake + Al₂O₃ + SnO₂ + TiO₂ + TiO₂/Fe₂O₃ + Fe₂O₃ + SiO₂
glass flake + SiO₂ + Al₂O₃ + SnO₂ + TiO₂ + TiO₂/Fe₂O₃ + Fe₂O₃ + SiO₂
Al₂O₃ flake + SiO₂ + SnO₂ + TiO₂ + Fe₂O₃ + SiO₂
Al₂O₃ flake + Al₂O₃ + SnO₂ + TiO₂ + Fe₂O₃ + SiO₂
Al₂O₃ flake + SiO₂ + Al₂O₃ + SnO₂ + TiO₂ + Fe₂O₃ + SiO₂
Al₂O₃ flake + SiO₂ + SnO₂ + TiO₂ + TiO₂/Fe₂O₃ + Fe₂O₃ + SiO₂
Al₂O₃ flake + Al₂O₃ + SnO₂ + TiO₂ + TiO₂/Fe₂O₃ + Fe₂O₃ + SiO₂
Al₂O₃ flake + SiO₂ + Al₂O₃ + SnO₂ + TiO₂ + TiO₂/Fe₂O₃ + Fe₂O₃ + SiO₂.

13. Process for the preparation of the pigments according to one or more of Claims 1 to 12, **characterised in that** the coating of the substrates is carried out by wet-chemical methods by hydrolytic decomposition of metal salts in aqueous medium or in a fluidised-bed reactor by gas-phase coating.

14. Use of the pigments according to one or more of Claims 1 to 12 in paints, coatings, automotive paints, powder coatings, printing inks, security printing inks, plastics, ceramic materials, glasses, paper, in toners for electrophotographic printing processes, in seed, in greenhouse sheeting and tarpaulins, as absorbers in the laser marking of paper and plastics, in cosmetic formulations, for the preparation of pigment pastes with water, organic and/or aqueous solvents, for the preparation of pigment preparations and dry preparations, for the mass colouring of foods, for the colouring of coatings of food products and pharmaceutical products and in documents of value.

15. Use of the pigments according to Claim 14 in lipsticks, lip gloss, rouge, eyeliner, eye shadow, (volume) mascara, nail varnishes, care creams, day creams, night creams, body lotions, cleansing milk, body powders, hair gels, hair lacquer, hair masks, hair rinses, hair shampoos, shower gels, shower oils, bath oils, sunscreen, pre-sun and after-sun preparations, tanning lotions, tanning sprays, make-ups, lotions, soaps, bath salts, toothpaste, facemasks, compact powders, loose powders and gels.

16. Formulations comprising the pigment according to one or more of Claims 1 to 12.

17. Formulations according to Claim 16 comprising the pigment according to one or more of Claims 1 to 12 and at least one constituent selected from the group of the absorbents, adstringents, antimicrobial substances, antioxidants, antiperspirants, antifoams, antidandruff active compounds, antistatics, binders, biological additives, bleaches, chelating agents, deodorisers, emollients, emulsifiers, emulsion stabilisers, dyes, humectants, film formers, fillers, fragrances, flavours, insect repellents, preservatives, anticorrosion agents, cosmetic oils, solvents, oxidants, vegetable constituents, buffer substances, reducing agents, surfactants, propellant gases, opacifiers, UV filters and UV absorbers, denaturing agents, viscosity regulators, perfume and vitamins.

## Revendications

1. Pigments basés sur des substrats sous forme de flocons à multiples revêtements, **caractérisés en ce que** :
(A0) en option, une couche de SiO₂ ;
(A) un revêtement incolore qui présente un indice de réfraction n < 1,8, où la couche (A) n'est pas chimiquement identique à la couche (A0) ;
(B) un revêtement incolore qui présente un indice de réfraction n ≥ 1,8, où la couche (B) présente une épaisseur de 1-15 nm ;
(C) un revêtement incolore qui présente un indice de réfraction n ≥ 1,8, où la couche (C) n'est pas chimiquement identique à la couche (B) ;
(D) un revêtement incolore qui présente un indice de réfraction n ≥ 1,8
et en option
(E) une couche de protection externe ;
sont sur le substrat.

2. Pigments selon la revendication 1, **caractérisés en ce que** les substrats sous forme de flocons sont du mica naturel ou synthétique, du talc, du kaolin, des oxydes de fer ou des oxydes d'aluminium sous forme de flocons, des flocons de verre, des flocons de SiO₂, des flocons de TiO₂, des flocons de graphite, des flocons synthétiques exempts de support, du nitrure de titane, du siliciure de titane, des polymères de cristaux liquides (LCP), des pigments holographiques, du BiOCl et des oxydes mélangés sous forme de flocons ou des mélanges afférents.

3. Pigments selon la revendication 1 ou 2, **caractérisés en ce que** les substrats sous forme de flocons sont des flocons de verre, des flocons de mica naturel, des flocons de mica synthétique ou des flocons d'oxyde d'aluminium.

4. Pigments selon une ou plusieurs des revendications 1 à 3, **caractérisés en ce que** les substrats sous forme de flocons sont des flocons de verre ou des flocons de mica synthétique

5. Pigments selon une ou plusieurs des revendications 1 à 4, **caractérisés en ce que** les flocons de verre sont constitués par du verre à vitres, du verre de borosilicate d'aluminium et de calcium, du verre A, du verre C, du verre E, du verre ECR, du verre Duran®, du verre d'équipements de laboratoire.

6. Pigments selon une ou plusieurs des revendications 1 à 5, **caractérisés en ce que** les flocons de verre sont constitués par du verre de borosilicate d'aluminium et de calcium, du verre C, du verre E ou du verre ECR.

7. Pigments selon une ou plusieurs des revendications 1 à 6, **caractérisés en ce que** la couche (A0) est dopée avec des particules de noir de charbon, des particules de métal et/ou des pigments colorés.

8. Pigments selon une ou plusieurs des revendications 1 à 7, **caractérisés en ce que** les couches (A) à (D) sont constituées par des oxydes.

9. Pigments selon une ou plusieurs des revendications 1 à 8, **caractérisés en ce que** les oxydes sont sélectionnés parmi le groupe qui est constitué par Al₂O₃, TiO₂, ZrO₂, SnO₂, ZnO, Ce₂O₃, Fe₂O₃, Fe₃O₄, FeOOH, Cr₂O₃, CoO, Co₃O₄, SiO₂, VO₂, V₂O₃, NiO, les sous-oxydes de titane ou des mélanges afférents.

10. Pigments selon une ou plusieurs des revendications 1 à 9, **caractérisés en ce qu'**ils comportent une couche de protection externe (E) pour augmenter la stabilité à la lumière, à la température et aux conditions météorologiques.

11. Pigments selon une ou plusieurs des revendications 1 à 10, **caractérisés en ce qu'**une autre intercouche (C0) qui est constituée par les composants des couches (C) et (D) est présente entre les couches (C) et (D).

12. Pigments selon une ou plusieurs des revendications 1 à 11, **caractérisés en ce que** les pigments présentent la structure de couches qui suit :
flocon de mica (naturel) + SiO₂ + SnO₂ + TiO₂ + Fe₂O₃
flocon de mica (naturel) + Al₂O₃ + SnO₂ + TiO₂ + Fe₂O₃
flocon de mica (naturel) + SiO₂ + Al₂O₃ + SnO₂ + TiO₂ + Fe₂O₃
flocon de mica (synthétique) + SiO₂ + SnO₂ + TiO₂ + Fe₂O₃
flocon de mica (synthétique) + Al₂O₃ + SnO₂ + TiO₂ + Fe₂O₃
flocon de mica (synthétique) + SiO₂ + Al₂O₃ + SnO₂ + TiO₂ + Fe₂O₃
flocon de mica (naturel) + SiO₂ + SnO₂ + TiO₂ + TiOz/Fe₂O₃ + Fe₂O₃
flocon de mica (naturel) + Al₂O₃ + SnO₂ + TiO₂ + TiO₂/Fe₂O₃ + Fe₂O₃
flocon de mica (naturel) + SiO₂ + Al₂O₃ + SnO₂ + TiO₂ + TiO₂/Fe₂O₃ + Fe₂O₃
flocon de mica (synthétique) + SiO₂ + SnO₂ + TiO₂ + TiO₂/Fe₂O₃ + Fe₂O₃
flocon de mica (synthétique) + Al₂O₃ + SnO₂ + TiO₂ + TiO₂/Fe₂O₃ + Fe₂O₃
flocon de mica (synthétique) + SiO₂ + Al₂O₃ + SnO₂ + TiO₂ + TiO₂/Fe₂O₃ + Fe₂O₃
flocon de verre + SiO₂ + SnO₂ + TiO₂ + Fe₂O₃
flocon de verre + Al₂O₃ + SnO₂ + TiO₂ + Fe₂O₃
flocon de verre + SiO₂ + Al₂O₃ + SnO₂ + TiO₂ + Fe₂O₃
flocon de verre + SiO₂ + SnO₂ + TiO₂ + TiO₂/Fe₂O₃ + Fe₂O₃
flocon de verre + Al₂O₃ + SnO₂ + TiO₂ + TiO₂/Fe₂O₃ + Fe₂O₃
flocon de verre + SiO₂ + Al₂O₃ + SnO₂ + TiO₂ + TiO₂/Fe₂O₃ + Fe₂O₃
flocon d'Al₂O₃ + SiO₂ + SnO₂ + TiO₂ + Fe₂O₃
flocon d'Al₂O₃ + Al₂O₃ + SnO₂ + TiO₂ + Fe₂O₃
flocon d'Al₂O₃ + SiO₂ + Al₂O₃ + SnO₂ + TiO₂ + Fe₂O₃
flocon d'Al₂O₃ + SiO₂ + SnO₂ + TiO₂ + TiO₂/Fe₂O₃ + Fe₂O₃
flocon d'Al₂O₃ + Al₂O₃ + SnO₂ + TiO₂ + TiO₂/Fe₂O₃ + Fe₂O₃
flocon d'Al₂O₃ + SiO₂ + Al₂O₃ + SnO₂ + TiO₂ + TiO₂/Fe₂O₃ + Fe₂O₃
flocon de mica (naturel) + SiO₂ + SnO₂ + TiO₂ + Fe₂O₃ + SiO₂
flocon de mica (naturel) + Al₂O₃ + SnO₂ + TiO₂ + Fe₂O₃ + SiO₂
flocon de mica (naturel) + SiO₂ + Al₂O₃ + SnO₂ + TiO₂ + Fe₂O₃ + SiO₂
flocon de mica (synthétique) + SiO₂ + SnO₂ + TiO₂ + Fe₂O₃ + SiO₂
flocon de mica (synthétique) + Al₂O₃ + SnO₂ + TiO₂ + Fe₂O₃ + SiO₂
flocon de mica (synthétique) + SiO₂ + Al₂O₃ + SnO₂ + TiO₂ + Fe₂O₃ + SiO₂
flocon de mica (naturel) + SiO₂ + SnO₂ + TiO₂ + TiO₂/Fe₂O₃ + Fe₂O₃ + SiO₂
flocon de mica (naturel) + Al₂O₃ + SnO₂ + TiO₂ + TiO₂/Fe₂O₃ + Fe₂O₃ +SiO₂
flocon de mica (naturel) + SiO₂ + Al₂O₃ + SnO₂ + TiO₂ + TiO₂/Fe₂O₃ + Fe₂O₃ + SiO₂
flocon de mica (synthétique) + SiO₂ + SnO₂ + TiO₂ + TiO₂/Fe₂O₃ + Fe₂O₃ + SiO₂
flocon de mica (synthétique) + Al₂O₃ + SnO₂ + TiO₂ + TiO₂/Fe₂O₃ + Fe₂O₃ + SiO₂
flocon de mica (synthétique) + SiO₂ + Al₂O₃ + SnO₂ + TiO₂ + TiO₂/Fe₂O₃ + Fe₂O₃ + SiO₂
flocon de verre + SiO₂ + SnO₂ + TiO₂ + Fe₂O₃ + SiO₂
flocon de verre + Al₂O₃ + SnO₂ + TiO₂ + Fe₂O₃ + SiO₂
flocon de verre + SiO₂ + Al₂O₃ + SnO₂ + TiO₂ + Fe₂O₃ + SiO₂
flocon de verre + SiO₂ + SnO₂ + TiO₂ + TiO₂/Fe₂O₃ + Fe₂O₃ + SiO₂
flocon de verre + Al₂O₃ + SnO₂ + TiO₂ + TiO₂/Fe₂O₃ + Fe₂O₃ + SiO₂
flocon de verre + SiO₂ + Al₂O₃ + SnO₂ + TiO₂ + TiO₂/Fe₂O₃ + Fe₂O₃ + SiO₂
flocon d'Al₂O₃ + SiO₂ + SnO₂ + TiO₂ + Fe₂O₃ + SiO₂
flocon d'Al₂O₃ + Al₂O₃ + SnO₂ + TiO₂ + Fe₂O₃ + SiO₂
flocon d'Al₂O₃ + SiO₂ + Al₂O₃ + SnO₂ + TiO₂ + Fe₂O₃ + SiO₂
flocon d'Al₂O₃ + SiO₂ + SnO₂ + TiO₂ + TiO₂/Fe₂O₃ + Fe₂O₃ + SiO₂
flocon d'Al₂O₃ + Al₂O₃ + SnO₂ + TiO₂ + TiO₂/Fe₂O₃ + Fe₂O₃ + SiO₂
flocon d'Al₂O₃ + SiO₂ + Al₂O₃ + SnO₂ + TiO₂ + TiO₂/Fe₂O₃ + Fe₂O₃ + SiO₂.

13. Procédé pour la préparation des pigments selon une ou plusieurs des revendications 1 à 12, **caractérisé en ce que** le revêtement des substrats est mis en oeuvre au moyen de procédés chimiques en phase humide par décomposition hydrolitique de sels de métaux dans un milieu aqueux ou dans un réacteur à lit fluidisé au moyen d'un revêtement en phase gazeuse .

14. Utilisation des pigments selon une ou plusieurs des revendications 1 à 12 dans des peintures, des revêtements, des peintures d'automobiles, des revêtements pulvérulents, des encres d'impression, des encres d'impression de sécurité, des matières plastiques, des matériaux de céramique, des verres, du papier, dans des toners pour des processus d'impression électrophotographique, dans des graines ou semences, dans des feuilles de plastique et des bâches de serres, en tant qu'absorbeurs au niveau du marquage au laser du papier et des matières plastiques, dans des formulations cosmétiques, pour la préparation de pâtes pigmentaires avec de l'eau, dans les solvants organiques et/ou aqueux, pour la préparation de préparations de pigments et de préparations sèches, pour la coloration en masse des aliments, pour la coloration des revêtements ou enrobages de produits alimentaires et de produits pharmaceutiques ainsi qu'au niveau de documents de valeurs.

15. Utilisation des pigments selon la revendication 14 dans les rouges à lèvres, le brillant à lèvres, le rouge, l'eyeliner, le fard à paupières, le mascara (volume), les vernis à ongles, les crèmes de soins, les crèmes de jour, les crèmes de nuit, les lotions pour le corps, le lait démaquillant, les poudres pour le corps, les gels pour les cheveux, la laque, les masques capillaires, les rinçages capillaires, les shampooings pour les cheveux, les gels pour la douche, les huiles pour la douche, les huiles pour le bain, les écrans solaires, les préparations pour soins avant exposition au soleil et après exposition soleil, les lotions auto-bronzantes, les sprays de bronzage, les produits pour maquillage, les lotions, les savons, les sels de bain, la pâte dentifrice, les masques faciaux, les poudres compactes, les poudres libres et les gels.

16. Formulations comprenant le pigment selon une ou plusieurs des revendications 1 à 12.

17. Formulations selon la revendication 16 comprenant le pigment selon une ou plusieurs des revendications 1 à 12 et au moins un constituant qui est sélectionné parmi le groupe qui est constitué par les absorbants, les astringents, les substances antimicrobiennes, les antioxydants, les anti-transpirants, les anti-moussants, les composés actifs antipelliculaires, les agents antistatiques, les liants, les additifs biologiques, les agents de blanchiment, les agents chélatants, les désodorisants, les émollients, les émulsifiants, les stabiliseurs d'émulsion, les colorants, les agents humidifiants, les agents filmogènes, les matières de remplissage, les fragrances, les arômes, les insectifuges, les conservateurs, les agents anticorrosion, les huiles cosmétiques, les solvants, les oxydants, les composants végétaux, les substances tampon, les agents de réduction, les agents tensioactifs, les gaz propulseurs, les opacifiants, les filtres UV et les absorbeurs d'UV, les agents de dénaturation, les régulateurs de viscosité, les parfums et les vitamines.
